# EUROPEAN PATENT SPECIFICATION

(11) **EP 4 609 399 B1**
(45) Date of publication and mention of the grant of the patent: **07.10.2026**
(21) Application number: 23798399.4
(22) Date of filing: 25.10.2023
(51) Int. Cl.: G16H 20/70, G16H 50/20, G16H 50/70

(54) **METHOD TO DETERMINE OPTIMAL PROGRESSION OF A COGNITIVE PROCESS REHABILITATION TASK**
VERFAHREN ZUR BESTIMMUNG DES OPTIMALEN FORTSCHREITENS EINER REHABILITATIONSAUFGABE FÜR KOGNITIVE PROZESSE
PROCÉDÉ POUR DÉTERMINER LA PROGRESSION OPTIMALE D'UNE TÂCHE DE RÉÉDUCATION DE PROCESSUS COGNITIF

(30) Priority: 25.10.2022 EP 22383029
(43) Date of publication of application: 03.09.2025
(73) Proprietor: Sincrolab, S.L., 28033 Madrid (ES)
(72) Inventor: DE RAMÓN BURGOS, Ignacio, 28033 Madrid (ES)
(74) Representative: Hoffmann Eitle
(86) International application number: PCT/EP2023/079849
(87) International publication number: WO 2024/089137

(56) References cited:
- WO-A1-2022/086454
- ANONYMOUS: "k-nearest neighbors algorithm - Wikipedia", 4 June 2022 (2022-06-04), XP093008901, Retrieved from the Internet <URL:https://en.wikipedia.org/w/index.php?title=K-nearest_neighbors_algorithm&oldid=1091525121> [retrieved on 20221216]

## Description

### Technical field of the invention

The present invention belongs to the field of neurology in particular, to a computer-implemented method for determining the optimal parameters for the progression of a cognitive rehabilitation task of a user in an adaptative and personalised way, and a computer program of the same.

### Background of the invention

Neurocognitive deficits may arise through the vital cycle of a person, at the beginning of the neurologic development, during childhood, teenage, adult age, and/or at the neurological decline through the middle age or the old age.

These neurocognitive deficits may be treated through cognitive training treatment, wherein the user exercises certain cognitive process, stimulating the associated areas of the brain and promoting the recovery of function in those associated areas.

The main problem current cognitive training treatment techniques deal with, is finding, in each moment, the best treatment for a patient. The treatment shall be personalised. Such that it is adapted to the pathology and profile of the patient; and adaptative, such that it evolves according to the needs of the patient while still being effective.

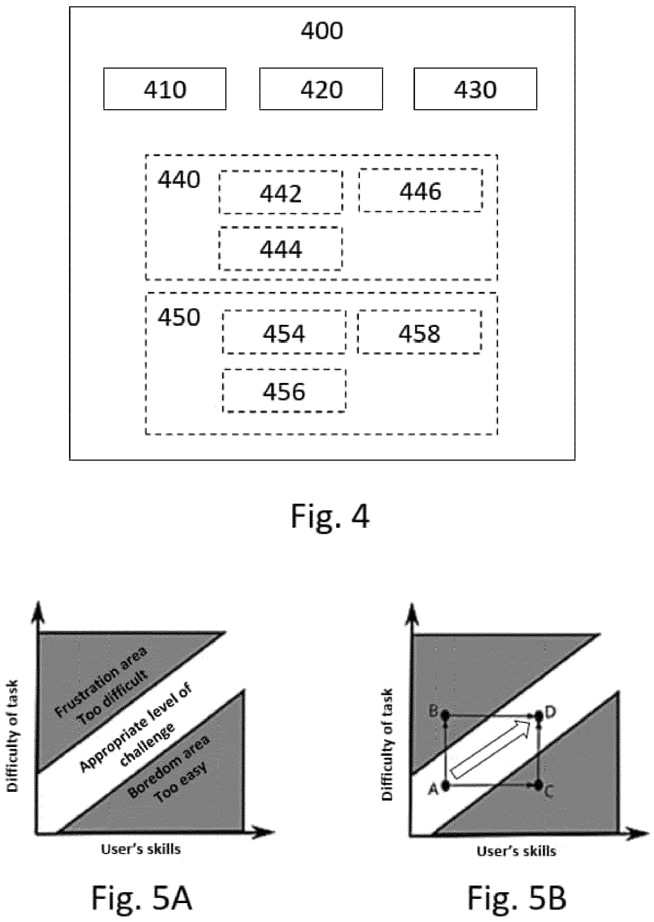

The appropriate and personalised development of the difficulty of the treatment can be achieved with the method dynamic game difficulty, balancing dynamic difficulty adjustment (DDA) or dynamic game balancing (DGB), which basically consists of adjusting the degree of difficulty of a video game dynamically, so that the user experiences the highest possible degree of satisfaction, as shown with respect to Figs. 5A and 5B. Fig. 5A shows the increase in difficulty of a task in relation to the increase in the user's skills. It can be seen that there are two well-defined areas to be avoided: the area of excessive difficulty or frustration, and the area of excessive ease or boredom. In the middle of the two areas there is a channel where the appropriate level of challenge is defined. Fig. 5B shows several possible example paths. In the ideal situation, a user in state A should move directly to state D (big arrow), but in practice transitions often occur by invading the frustration and boredom areas (points B and C respectively). The goal of most cognition training treatments based on video games is therefore to keep the user within that channel of satisfaction as their skills increase. In training, each of the jumps involves choosing a set of values for the variables that govern the games.

There is therefore a need in current cognitive training treatment when implementing this DDA or DGB methods of finding at each time point the set of values that lead the patient through the interior of this satisfaction channel. Also, the most convenient task for the patient may affect the cognitive treatment and how easy the DDA or DGB methods can be implemented. Therefore, there is also a need for a method to choose the most suitable tasks for a patient.

WO 2022/086454 A1 discloses a personalised cognitive digital therapeutic system which monitors the effect of therapy on individual patient and can provide valuable insights to the caregiver/clinician for better therapeutic decision making.

### Summary of the invention

The invention is defined by the appended claims.

### Brief description of the drawings

To enable a better understanding of the present disclosure, and to show how the present disclosure may be carried out, reference will now be made, by way of example only, to the accompanying schematic drawings, wherein:
Figure 1 shows a diagram of a method for determining the optimal parameters for the progression of a cognitive rehabilitation task of a user according to one or more embodiments of the invention.
Figure 2 shows a diagram of a method for selecting a subset of k cases from a set of past cases according to one or more embodiments of the invention.
Figure 3 shows a diagram of a method for determining a task to be performed by a user according to one or more embodiments of the invention.
Figure 4 shows a computer program product diagram according to one or more embodiments of the invention.
Figure 5A shows a graph for the appropriate level of challenge for a user.
Figure 5B shows several transition paths in cognition training treatments based on video games.
Figure 6 graphically illustrates the time intervals selected for the modelling process in accordance with one or various embodiments of the invention.
Figure 7 shows the distribution of the difference in performance of the last quartile vs. the baseline of an example according to one or more embodiments of the invention. The vertical line is the 1 standard deviation cut-off for improvement.
Figure 8 shows the distribution of number of training sessions per patient vs. improvement (0 no improvement, 1 improvement) of an example according to one or more embodiments of the invention
Figure 9 shows ROC and AUC plots of results on real patient data according to one or more embodiments of the invention.

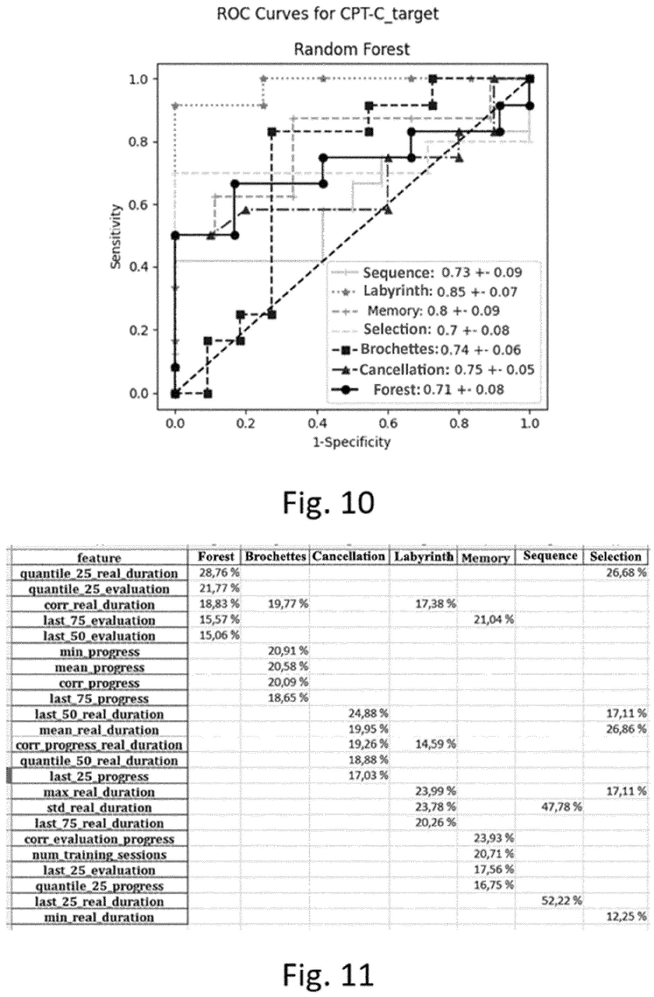

Figure 10 shows the predictive power (ROC curve) of measuring cognitive tasks in the Commissions Index in the CPT-III of an example according to one or more embodiments of the invention.
Figure 11 shows the most important features per cognitive task in the construction of the predictive model in percentage of an example according to one or more embodiments of the invention.

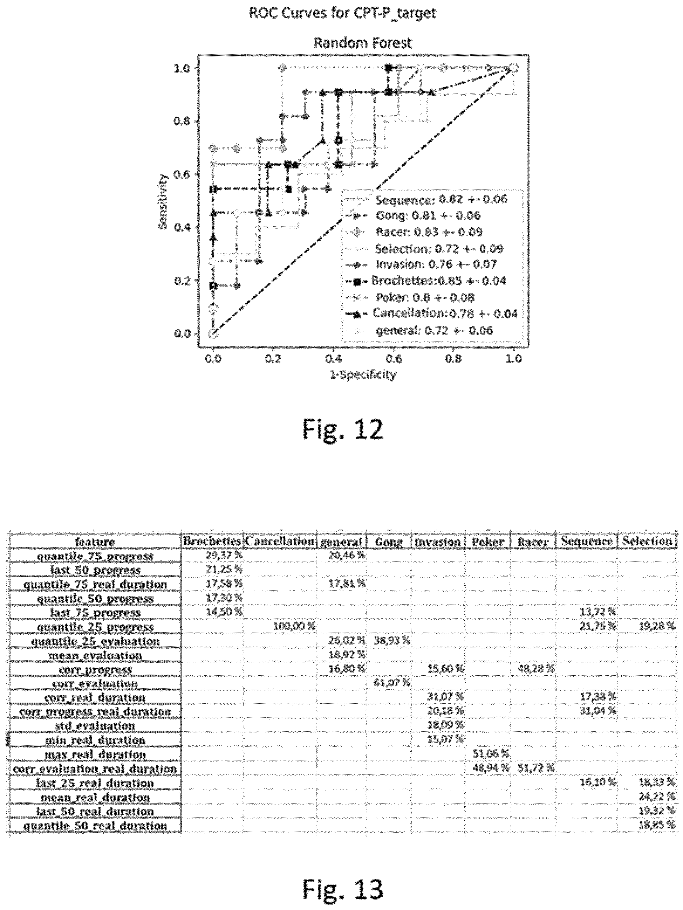

Figure 12 shows the predictive power (ROC curve) of measuring the cognitive tasks in the Perseverations index in the CPT-III of an example according to one or more embodiments of the invention.
Figure 13 shows the most important features per cognitive task in the construction of the predictive model in percentage of an example according to one or more embodiments of the invention.

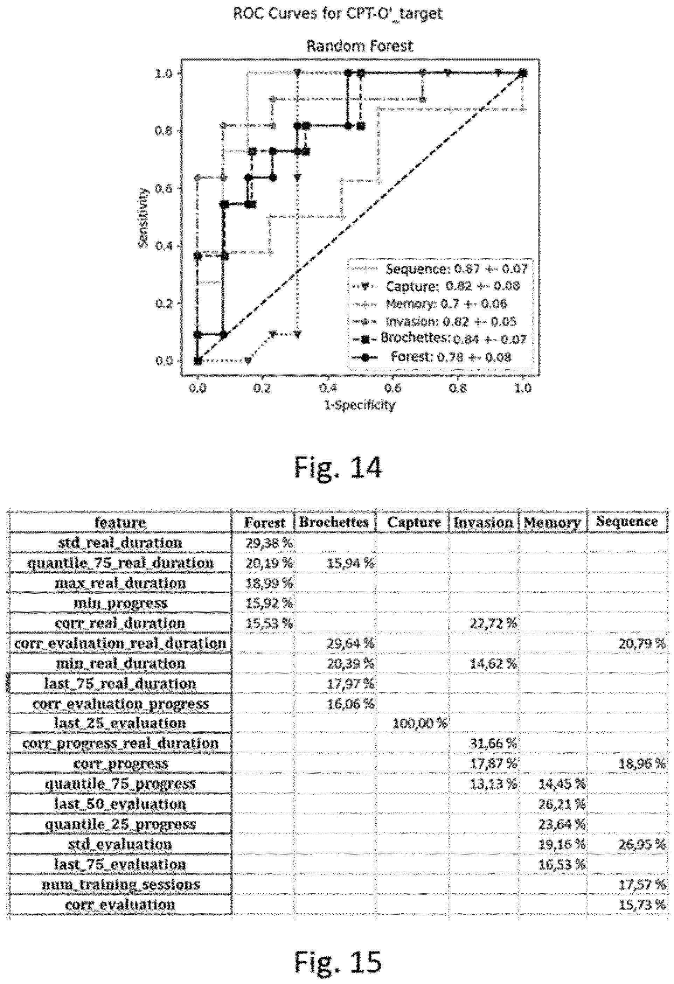

Figure 14 shows the predictive power (ROC curve) of measuring the cognitive tasks in the Omissions index in the CPT-III of an example according to one or more embodiments of the invention.
Figure 15 shows the most important features per cognitive task in the construction of the predictive model in percentage of an example according to one or more embodiments of the invention

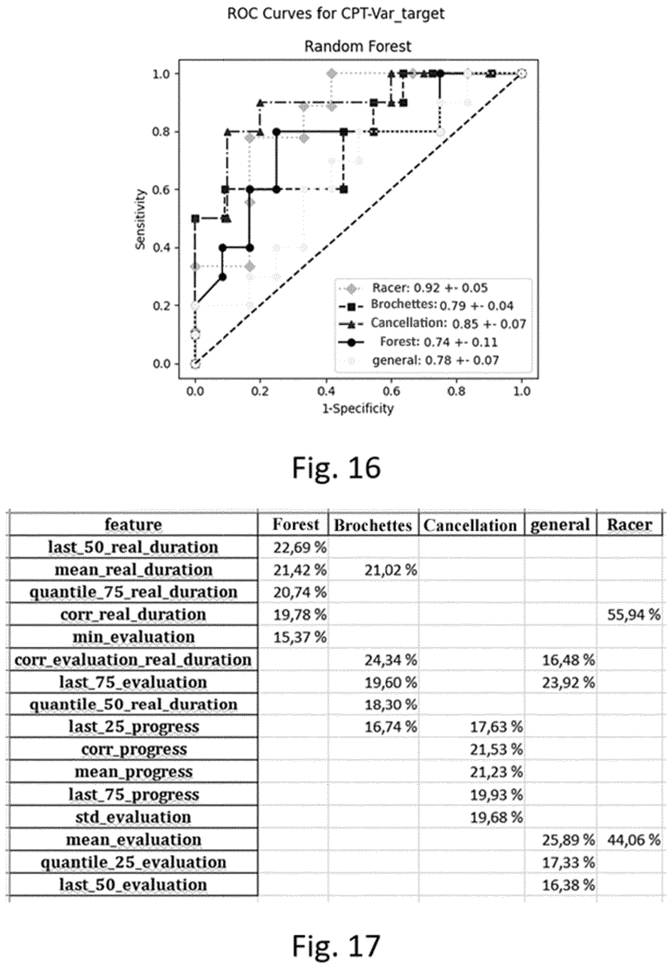

Figure 16 shows the predictive power (ROC curve) of measuring the cognitive tasks in the Response Variability index in the CPT-III of an example according to one or more embodiments of the invention.
Figure 17 shows the most important features per cognitive task in the construction of the predictive model in percentage of an example according to one or more embodiments of the invention.

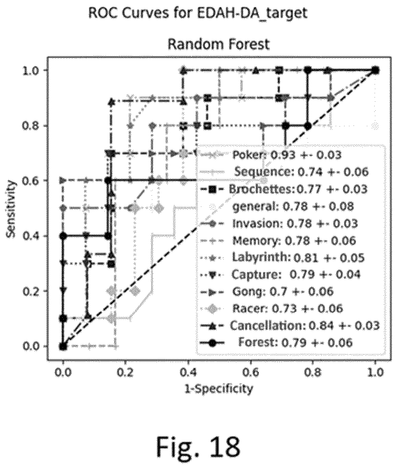

Figure 18 shows the predictive power (ROC curve) of measuring cognitive tasks in the clinical index of Attention Deficit (AD) in ADHD. of an example according to one or more embodiments of the invention

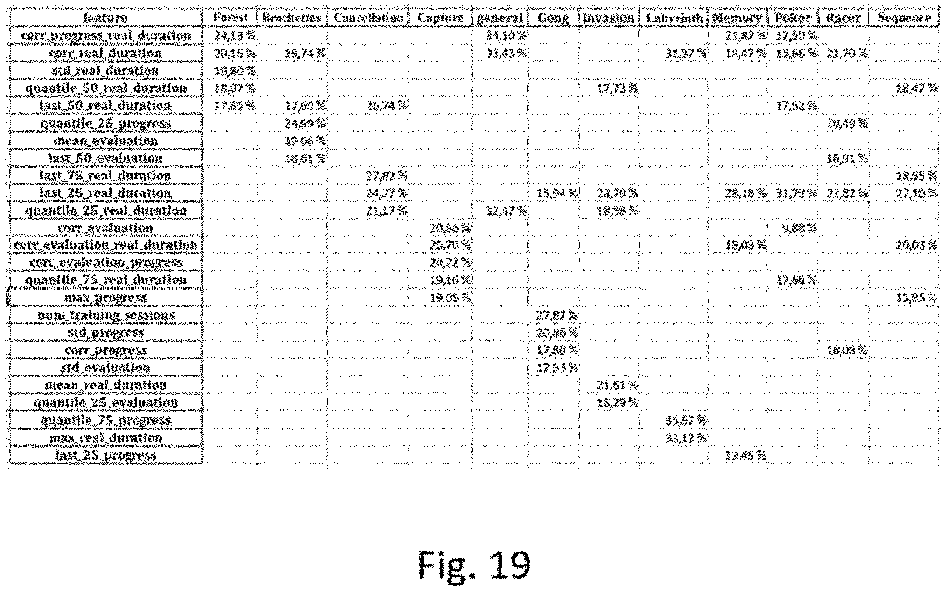

Figure 19 shows the most important features per cognitive task in the construction of the predictive model in percentage of an example according to one or more embodiments of the invention.

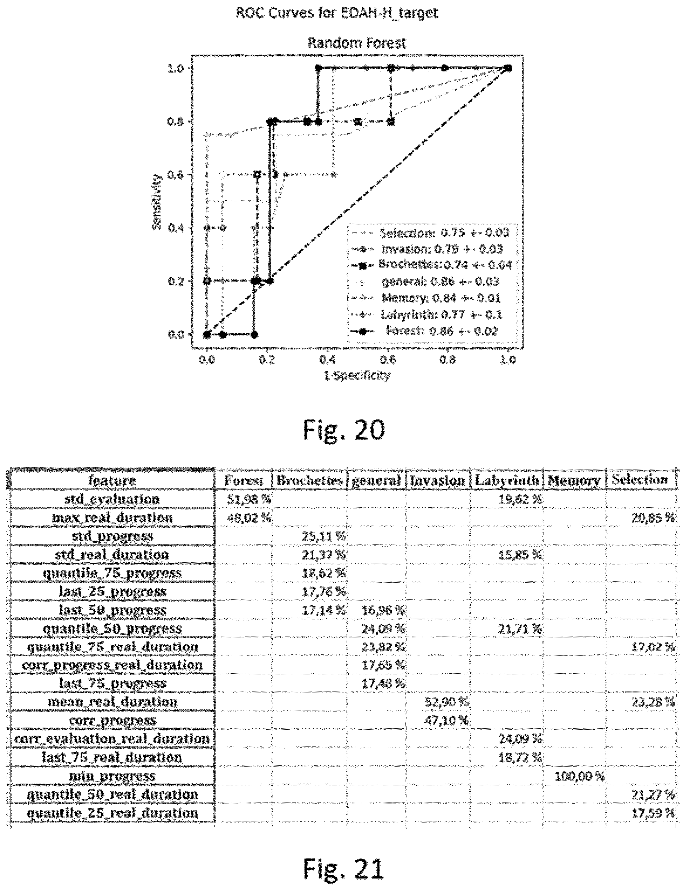

Figure 20 shows the predictive power (ROC curve) of measuring cognitive tasks in the clinical index of Hyperactivity (H) in ADHD of an example according to one or more embodiments of the invention.
Figure 21 shows the most important features per cognitive task in the construction of the predictive model in percentage of an example according to one or more embodiments of the invention.

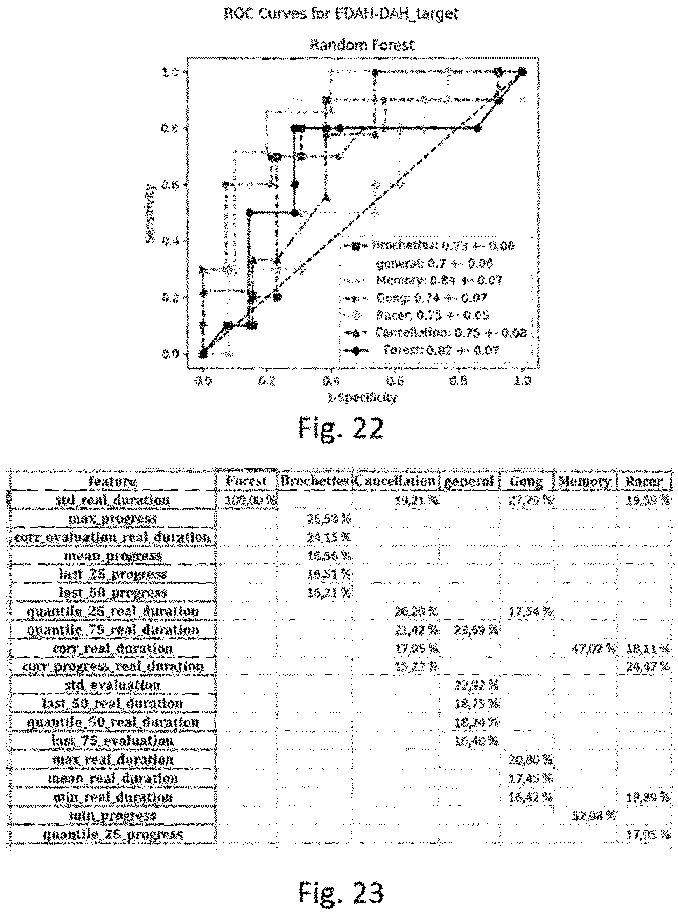

Figure 22 shows the predictive power (ROC curve) of measuring cognitive tasks in the clinical index of Attention Deficit and Hyperactivity (ADH) in ADHD of an example according to one or more embodiments of the invention.
Figure 23 shows the most important features per cognitive task in the construction of the predictive model in percentage of an example according to one or more embodiments of the invention.
Figure 24 shows the evaluation from the users on the level of difficulty provided by the method when 2 vs 4 distances are used as part of the CBR paradigm for the closest cases selection from Example 2 according to one or more embodiments of the invention.
Figure 25 shows a visualisation of the distribution of progress between the 2 vs 4 distances methods from Example 2 according to one or more embodiments of the invention.

### Description of the invention

### Definitions

As used herein and in the appended claims, the singular forms "a", "an" and "the" include plural referents unless the context clearly dictates otherwise. Also, the use of "or" means "and/or" unless stated otherwise. Similarly, "comprise," "comprises," "comprising" "include," "includes," and "including" are interchangeable and not intended to be limiting.

Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein.

The publications discussed above and throughout the text are provided solely for their disclosure prior to the filing date of the present application. Nothing herein is to be construed as an admission that the inventors are not entitled to antedate such disclosure by virtue of prior disclosure.

As used herein, the words or terms set forth below have the following definitions.

The term "cognitive process" refers to any of the mental functions assumed to be involved in the acquisition, storage, interpretation, manipulation, transformation, and use of knowledge. In the present invention, the cognitive process preferably encompass activities such as attention, perception, learning, and problem solving.

The term "cognitive profile" refers to the unit of information comprising a set of values from one or more cognitive processes of a user, that defines the user's pattern of relative strengths and difficulties across one or several cognitive domains. It identifies the cognitive state of the user, its functional capacities and affordances.

The term "distance" refers to a measure of dissimilarity between two comparable elements. In the present invention it is preferably understood as the difference between the values of the parameters of the task or the user, such as task definitions, task configurations, task results and user cognitive profile.

The term "sustained attention" refers to the attentional focus of a person on a task for a determined period of time, preferably wherein the period of time is an extended length of time.

The term "selective attention" refers to the attentional focus of a person on certain stimulus or stimuli in the environment among other stimuli. The terms "controlled attention", "directed attention", and "executive attention" may be used interchangeably.

The term "divided attention" refers to the attentional focus of a person on two or more channels of information at the same time, so that two or more tasks may be performed concurrently. It may involve the use of just one sense (e.g., hearing) or two or more senses (e.g., hearing and vision).

The term "processing speed" refers to the time it takes an person to do a mental task. In the present invention the processing speed is preferably computed as the speed in which a person can understand and react to the information they receive, whether it be visual (letters and numbers), auditory (language), or movement.

The term "decision making" refers to the cognitive process of choosing between two or more alternatives. Decision making process may range from relatively simple binary decisions (e.g., ordering a meal at a restaurant) to complex decisions (e.g., selecting a mate).

The term "inhibitory control" refers to the cognitive process that allows a person to inhibit their impulses and natural, habitual, or dominant behavioural responses to stimuli (i.e., learned prepotent responses) in order to select more appropriate behaviours that are consistent with one's goals. The term "response inhibition" may be used interchangeably. The term "cognitive flexibility" refers to the cognitive process of being capable of objective appraisal and appropriately flexible action. It is an indicator of the adaptability and fair-mindedness of a person.

The term "operative memory" refers to the set of cognitive processes that allow a person to store and manipulate temporary information and carry-out complex cognitive tasks like language comprehension, reading, learning, or reasoning. It is a type of short-term memory and the term "working memory" may be used interchangeably. The term "task definition" refers to the set of parameters that characterises each task. This may comprise, for example, the minimum and maximum values of the task variables. The task definition may therefore vary among tasks, as each task may comprise a set of different variables characterising it.

The term "task configuration" refers to the set of parameters that configures each task to be when executed. This may comprise, for example, the values assigned to the task variables.

### Description

A first aspect of the invention relates to a computer-implemented method for determining the optimal parameters for the progression of a cognitive process rehabilitation task of a user. The progression is adaptative and personalised to the user by taking into account previous performances of the user to determine the parameters to be established in each rehabilitation task performed.

The program and its modules may be stored in a local device memory (e.g. in the device executing the task), or it can be stored remotely, e.g. on a remote server connected communicatively to the device. Similarly, the program and its modules may be processed by a local processor (e.g. in the device executing the task), or it can be processed remotely, e.g., on a remote server connected communicatively to the device.

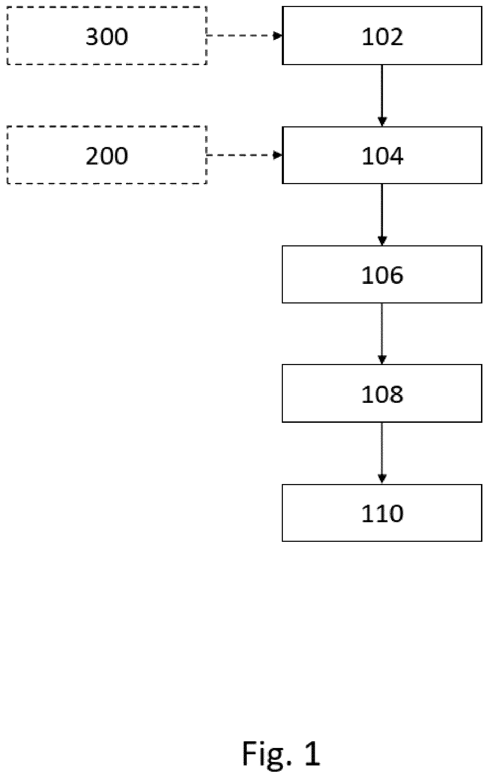

As shown with respect to Fig.1 the computer- implemented method comprises several steps. First, as shown in step 102, it comprises receiving a determined task to be performed by the user. This task is a cognitive process rehabilitation task, aimed at training and improving the performance of the subject carrying out such task in one or more cognitive processes. The task comprises modifiable parameters. These modifiable parameters affect the difficulty of the task, so they can be tuned to change the difficulty of performing the task. However, these parameters define a multidimensional tuning tool, which affect the difficulty of the task in a non-obvious way to each user.

The modifiable parameters comprise one or more parameters selected from: length of the task, exposure time, interstimulus time, n-back, modality, sessions, trials, % of distractors, % of targets, delay, signal, stop or type of change.

All these parameters may be modified to make an impact on the task difficulty. For example, the length of the task may increase the difficulty if made longer, as the user is required to train for an extended period of time which demands long-term concentration. The exposure time may be defined as the time a particular stimulus is provided to the user. Making this exposure time shorter, may, in some tasks increase the difficulty if, for example, the task involves retaining some information associated to such stimuli, like the colour or shape. The interstimulus time may be defined as the time elapsed between two stimuli. In some tasks a longer interstimulus time may make the task more difficult to be performed, as the memory is challenged, while in other tasks a shorter interstimulus time may make the task more difficult, for example by increasing the amount of information provided in a given time frame. The n-back parameter may be defined as the number of previous stimuli to be recalled by the user. In some tasks the bigger the n-back parameter, the more difficult the task is for the user, as the user is required to use more memory.

The modality may be defined as a main characteristic of the task that controls significant changes both in task difficulty and cognitive processes involved in the task. For example, a given task may be configured in two modalities such as visual and auditory modality. In the visual modality, the stimuli presented to the user are visual stimuli, while in the auditory modality, the stimuli presented to the user are auditory modality. Thus, changing from a visual modality to an auditory modality may increase the task difficulty by changing the cognitive processes involved in the task for a user with a cognitive provide with higher impairment in the auditory-related cognitive processes than in the visual-related cognitive processes, and vice versa.

The amount of sessions to be performed may increase the difficulty of the task if the amount is increased as the user is required to perform the task for a longer time and introducing the concentration and tiredness effect onto the task. In some other cases, it may increase the difficulty of the task if the amount is decreased as the user is provided with less attempts to improve the task. The amount of trials to be performed may increase the difficulty of the task if the amount is increased as the user is required to perform the task more times making it less attractive or more difficult to focus on. In some other cases, it may increase the difficulty of the task if the amount is decreased as the user is provided with less attempts to learn the task.

A higher % of distractors may increase the difficulty of the task, for example by challenging the selective attention of the user on a target. A higher % of targets may also increase the difficulty of the task by challenging the divided attention of the user.

The delay may be defined as the time elapsed before an interactive item of the task is shown to the user. appears in the task. Increasing the delay may for example challenge the operative memory and inhibitory control of the user, while a smaller delay may reduce the need for the user to recall the instructions or to avoid getting distracted by external factors.

Given a certain signal to react to, or a feedback signal, it may be modified to affect the task difficulty. For example a bigger, louder or brighter signal may facilitate the task while a smaller, quieter or duller signal may make noticing such signal more difficult, therefore challenging the attention. Similarly, a stop indication may be modified to for example, challenge the inhibitory control.

It is noted that all thee parameter may be modified in one or more aspects and that this modifications may affect in a different way to each user depending on the cognitive processes affected in such user, defining the user cognitive profile. Therefore there is not a straightforward way to tune the set of parameters to achieve an adaptative a personalised progression of the rehabilitation. The cognitive processes may be any cognitive process that may be defined in a user, as disclosed further below.

It is also noted that in some embodiments, the modifiable parameters may comprise any other parameters from the task not described herein that can be modified or tuned to affect the difficulty of the task. This parameters may depend on the particular task as each task may be defined to train a different cognitive process and therefore the modifiable parameters change across tasks.

As a non-limiting disclosure a set of cognitive processes to be rehabilitated are now disclosed. The cognitive process is one or more cognitive process selected from: sustained attention, selective attention, divided attention, processing speed, decision making, inhibitory control, cognitive flexibility and/or operative memory. The skilled person may note other cognitive processes to which the methodology herein described may be applied. **It is also noted, that** although the method is described as being directed for determining the optimal parameters for the progression of a cognitive process rehabilitation task of a user, in some embodiments the task may be a rehabilitation task from more than one cognitive process.

Secondly, as shown in step 104 of Fig. 1, the method comprises receiving a set of k cases from a set of past cases. Each case may be a unit of information comprising info on the user, the task performed including the values of its modifiable parameters and any other configuration such as if has been somewhat adapted to the user, and the results of the task indicating the user's ability when playing such task in such attempt.

The set of k cases may be any subset of cases withing the set of past cases, including all the past cases. Therefore, the k number of cases is not limited to any number of cases. Preferably, the k cases comprise at least 2 cases from the set of past cases, even more preferably at least 5 cases.

The k cases are selected using a case-based reasoning (CBR) paradigm. In the present invention the CBR paradigm may comprise any algorithm wherein the selection of the k cases from the set of past cases comprises taking into account the similarity of previous cases with the present attempt of the user. This may comprise comparing any information of the present case with the previous cases. It is noted that the CBR paradigm may be implemented using any traditional or artificial intelligence algorithms, including but not limited to artificial neural networks, random forest, SVM and naive Bayes classifiers that may optimize the CBR paradigm algorithm to ensure the provided k cases serve as good indicators of the desired progression of a cognitive process rehabilitation task. It is noted the skilled person may foresee implementing the CBR paradigm in other machine learning algorithms not described herein.

Thirdly, as shown in step 106 of Fig. 1, the method comprises averaging the values of the modifiable parameters of the set of k cases received in step 104. It is noted that in some embodiments, the k cases may comprise information on different tasks and/or different modifiable parameters, and in such cases, only the modifiable parameters to be tuned in the task to be performed may be averaged. However, in some embodiments, the other parameters may be considered to weight the effect of each case in the final average. It is also noted that in some embodiments, the average may be the arithmetical mean of each parameter across the k cases, while in other embodiments, each parameter may be weighted according to the importance of such case for the task to be performed. In some embodiments all the parameters may be weighted equally, while in other embodiments, each case may be weighted differently.

Fourthly, as shown in step 108 of Fig. 1, the method comprises determining the optimal parameters of the task as the averaged modifiable parameters of step 106. It is noted that as the k cases received in step 104 have been selected using a CBR paradigm algorithm, it comprises a set of cases which represent good indicators of parameters settings that provide the desired progression of a cognitive process rehabilitation task, validated by the result of the task. Therefore, a mean value of such parameters for all cases comprise a good representation of the optimal parameters for the progression of the cognitive process rehabilitation task of the user.

The optimal parameters may be further processed by an algorithm, such as an Al or machine learning algorithm, to be set within a range of values, to be updated in a certain way (for example by first exploring an ascending value and a descending value) and/or to be updated only when some conditions are met (for example to only update a certain parameter if other parameter is updated within a certain range or not updated at all). Advantageously, this allow the optimal parameters to consider further implications of the set of modifiable parameters that do not depend on the past cases, but to the task implications or the optimization technique defined in the algorithm determining the optimal parameters. Therefore, for a given set of averaged values of the modifiable parameter, the optimization algorithm may consider setting different optimal parameters depending on other inputs not associated to the case, like a set optimization methodology of the algorithm.

Fifthly and finally, as shown in step 110 of Fig. 1, the method comprises, once the task is performed with the optimal parameters determined in step 108, updating the set of past cases with the results of the task performed with the optimal parameters of step 108. This involves creating a new past case to add to the set of past cases, comprising the optimal parameters of step 108 and the user and task information such as the task definition, cognitive profile, task configuration and results of the training.

Advantageously, this last step provides the method with a way to make the progression adaptative and personalised to the user. By the provision of each past case of the user to the set of past cases, the next time the CBR paradigm is used to select the set of k cases the last case will be taken into account, and given the proximity of the last case and the new case, as it's the same user and task, it is highly probable that the last case will be comprised into the selected set of k cases, and thus in step 108 the modifiable parameters used in the last case will be used to determine the optimal parameters of step 106. This will happen for each time the user performs a cognitive process rehabilitation task, generating a new case that will be fed to the set of past cases. Therefore, the more the user performs a cognitive process rehabilitation task, the more cases for such used will be comprised in the set of past cases, and the more information from the user may be used to optimize the modifiable parameters for the progression of a cognitive process rehabilitation in an adaptative and personalised way.

As mentioned above and as it will be explained further below, the determination of the optimal parameters from the modifiable parameters of the set of k past cases may be done in different ways. The performance of the past case, defined by the results of the cognitive process rehabilitation task of the past case may be taken into account to make each case have more or less weight on the determination of the optimal parameters of the task. For example the modifiable parameters of a case with bad results may be taken less into account that the modifiable parameters of a case with good results. Thus it is adaptative, as if some parameters result in bad task results for that user, the next iteration those parameters will be taken less into account, i.e. less weighted when determining the optimal parameters. It is also personalised, as not only the progression depends on the results, but the specific progression of the user under determined conditions is taken into account for future cases. Therefore the parameters for the progression of a cognitive process rehabilitation task of a user, are chosen for an adaptative and personalised progression of the same.

It is noted that in some embodiments, the computer-implemented method may be executed locally (e.g. in the device executing the task), while in other embodiments, the computer-implemented method may be executed remotely (e.g., on a remote server connected communicatively to the device). It is also possible that some steps of the method are executed locally, while other are executed remotely, such as those requiring more computational power, for example, when using Al or machine learning algorithms to implement any of the CBR paradigms.

In a preferred embodiment of the first aspect of the invention, the received k cases are the closest k cases to the user's new case and are selected using a case-based reasoning (CBR) paradigm which comprises computing the distance between each case c_{I} of the set of past cases and the last case c_{N}. The distance between cases is defined as an arbitrary value defining the difference between each two cases I and N, wherein one or more parameters of each case is used to compute such distance.

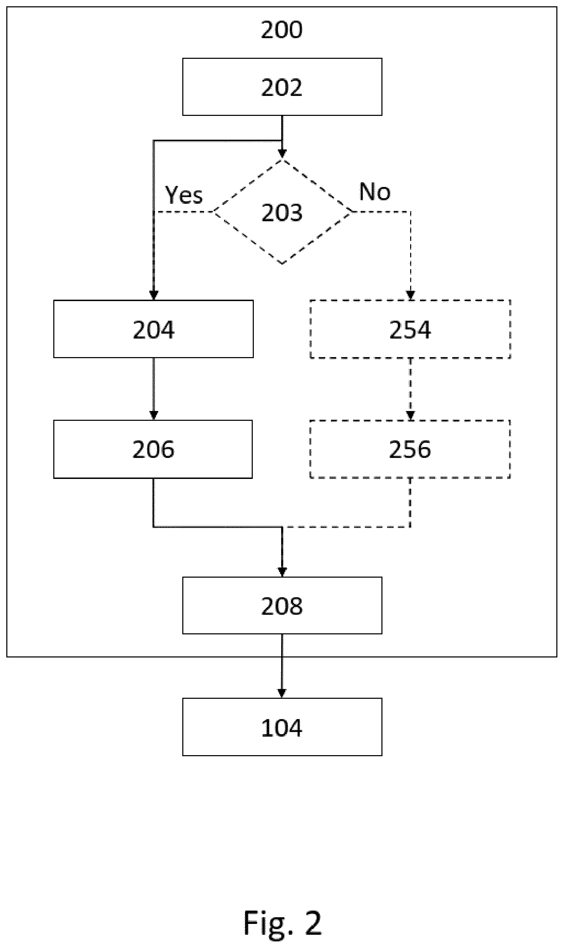

As shown with respect to Figs. 1 and 2, in a further preferred embodiment the closest k cases to the user's new case are determined by a computer-implemented method 200 that comprises several steps. It first comprises the step 202 of receiving a set of past cases. These past cases are cases obtained according to the present methodology, wherein each case is a unit of information that may comprise info on the user, the task performed including the values of its modifiable parameters and any other configuration such as if has been somewhat adapted to the user, and the results of the task indicating the user's ability when playing such task in such attempt. Then, in step 204, for each of the past cases Ci, the distance with the new case C_{N} is computed.

It is noted that as the distance defines the difference between two cases, the k closest cases to the user's new case would therefore be the k cases with the smallest distance to the new case c_{N}. The distance arbitrary value may be any value that may be directly correlated with the difference between two cases. Preferably, the distance arbitrary value may be normalized between 0 and 1.

Each case comprises a set of parameters that defines it, such as the task definition, the cognitive profile of the user, the task configuration and/or the task results. Each of these parameters may comprise one or more variables defining it. For example the task configuration may comprise one or more of length of the task, exposure time, interstimulus time, n-back, modality, size, sessions, trials, % of distractors, % of targets, delay, signal, stop, change or type of change. Also the results may comprise one or more or success rate or fail rate. Therefore, the difference in each of these variables between two cases may be used to define a distance between the cases.

For example, for two cases each of the variables may define a dimension and the parameter may be defined as a vector represented in such dimensional space. The distance between the vectors of cases cᵢ and c_{N} may be therefore used as a distance value for such parameter.

Once the distance for all the past cases with the new case has been computed, in step 206 the similarity of each case with the new case is computed, based on the distance of each past case with the new case computed in step 204. The similarity may be simply computed as an inverse function of the distance or may comprise a more complex function that considers more information of each case to determine the similarity of each past case with the new case. This may comprise for example a machine learning or artificial intelligence algorithm that uses the distance as an input to compute the similarity.

Finally, once the similarity of each past case with the new case is computed, in the step 208 the set of k closest past cases may be defined as the set of k cases with the most similarity with the new case. In some embodiments, some control rules, such as a minimum number of different users may be applied to avoid overfitting the selection algorithm.

Once the set of k closest cases are selected, they are provided to the computer-implemented method for determining the optimal parameters for the progression of a cognitive process rehabilitation task of a user in step 104. In some embodiments, the set of k cases provided onto step 104 may be limited according to the requirements of the method, for example, by selecting a subset of the set of k cases.

In another preferred embodiment, the received k cases are the closest k cases to the user's new case and are selected using a case-based reasoning (CBR) paradigm which comprises computing the distance between each case cᵢ of the set of past cases and the new case c_{N}.

As shown again with respect to Fig. 2, the method may comprise the step 202 of receiving a set of past cases. Then, in step 204, for each of the past cases Ci, the distance with the new case CN is computed as: ${d}_{\left({c}_{i}, {c}_{N}\right)} = {d}_{{TDef}_{i , N}} + {d}_{{Prof}_{i , N}} + {d}_{{Conf}_{i , N}} + {d}_{{Res}_{i , N}}$ wherein *d_{TDef_{í,N}}* is the distance between the task definitions of cases i and N, *d_{Prof_{í,N}}* is the distance between the cognitive profiles of the users of cases i and N, *d_{Conf_{í,N}}* is the distance between the tasks configurations of the cases i and N, and *d_{Res_{í,N}}* is the distance between the results of cases i and N. Therefore, the distance between cases is computed as the sum of differences between the task definition, the cognitive profile of the user, the task configuration and the task results.

Advantageously, as shown in Example 1, computing using a set of closest k cases selected using a CBR paradigm according to a distance between each case cᵢ of the set of past cases and the new case c_{N}, wherein such distance is computed as the sum of differences between the task definition, the cognitive profile of the user, the task configuration and the task results has shown ensures the computer-implemented method herein presented provides a new case cN with a clinical validated rehabilitation. Particularly, the method for determining the optimal parameters for the progression of a cognitive process rehabilitation task of a user, wherein the progression is adaptative and personalised according to this embodiment, ensures the rehabilitation task determined has CPT-III equivalent results. The Conners Continuous Performance Test (CPT-III; Conners, 2014) is a computerized continuous performance task which measures four dimensions of Inattentiveness, Impulsivity, Sustained Attention and Vigilance. The task is composed of 360 trials, grouped by 6 blocks with variable inter-stimulus intervals (ms) for each block in which stimuli targets are all the letters except the letter X that is the non-target. Participants had to press the spacebar each time a target letter appeared on the screen and inhibit this response when a non-target was shown. Each dimension (inattentiveness, impulsivity, sustained attention and vigilance) includes specific index as follows:
- Inattentiveness: commissions (C), detectability (CPT_d), omissions (CPT_O), hit reaction time (CPT_HRT), standard deviation of HRT (CPT_HRTSD), response variability (CPT_Var);
- Impulsivity: commissions (C), HRT and preservations (CPT_P)
- Sustained Attention: HRT block change, omissions by block (CPT_HRT and CPT_O);
- Vigilance: HRT inter-stimulus (CPT_HRTISI), Interval (ISI) change and omissions by ISI

It is also noted, that as shown in Example 2, the use of a distance between cases comprising the 4 distances above defined (*d_{TDef_{i,N}}, d_{Prof_{i,N}}, d_{Conf_{i,N}} and d_{Res_{í,N}}*) has a significant impact on the personalisation and adaptability of the cases to the user when compared to only using the distance between the tasks definitions *d_{TDef_{i,N}}* and the distance between the cognitive profiles of the patients *d_{Prof_{i,N}},* which implies a better treatment outcome.

According to some embodiments, each of these parameters of each case may be weighted differently to modify the effect each of these case parameters have on the definition of the distance between cases d(cᵢ, c_{N}). For example, it may be determined that the task results may have more impact on the distance between two cases at a determined moment, therefore, the *d_{Res_{í,N}}* may be scaled by a factor of, for example, 1.5 to have a higher impact on the distance d(cᵢ, c_{N}).

The distance between task definitions, cognitive profiles, task configurations and results is defined as an arbitrary value defining the difference between each two task definitions, cognitive profiles, task configurations and results, respectively, wherein one or more parameters of each task definition, cognitive profile, task configuration or result is used to compute such distance, respectively.

Each of these parameters may comprise one or more variables defining it. For example the task configuration may comprise one or more of length of the task, exposure time, interstimulus time, n-back, modality, size, sessions, trials, % of distractors, % of targets, delay, signal, stop, change or type of change. Also the results may comprise one or more or success rate or fail rate. Therefore, the difference in each of these variables between two cases may be used to define a distance between the task configurations and a distance between the task results, which in turn are used to compute the distance between the cases I and N. For example, for two cases each of the variables may define a dimension and the parameter may be defined as a vector represented in such dimensional space. The distance between the vectors of cases cᵢ and c_{N} may be therefore used as a distance value for such parameter.

As an exemplary embodiment, given a cognitive profile defined by the user's sustained attention, selective attention and divided attention impairment, wherein the user of interest has a sustained attention impairment of 0.1, a selective attention impairment of 0.7 and a divided attention impairment of 0.3, and a past user wherein the past user has a sustained attention impairment of 0.3, a selective attention impairment of 0.7 and a divided attention impairment of 0.2, the new user cognitive profile can be represented as the vector [0.1, 0.7, 0.3] and the past user cognitive profile can be represented as the vector [0.3, 0.7, 0.2]. Therefore, the distance between the cognitive profiles may be defined as the distance between the vectors [0.1, 0.7, 0.3] and [0.3, 0.7, 0.2].

As above explained, once the distance for all the past cases with the new case has been computed, in step 206 the similarity of each case with the new case is computed, based on the distance of each past case with the new case computed in step 204. Then, once the similarity of each past case with the new case is computed, the set of k closest past cases may be defined as the set of k cases with the most similarity with the new case. Finally, the set of k closest cases are provided to the computer-implemented method for determining the optimal parameters for the progression of a cognitive process rehabilitation task of a user in step 104. The same possible and alternative embodiments directed for each of the steps 206, 208 and 104 as described above regarding the previous preferred embodiment may be applied to this preferred embodiment.

In a further preferred embodiment, the distance between the task definitions of cases i and N computed in step 204 is computed as: ${d}_{TDef \left(i, N\right)} = \frac{1}{{D}_{N}} {\sum }_{∀ TDef} \left|{TDef}_{i}-{TDef}_{N}\right|$

*TDefᵢ* and *TDef_{N}* are the normalized coefficients ∈ [0,1] indicating the task definition parameter in each case i and N, respectively and D_{N} is a normalizing factor computed as the sum of max differences between the TDef values.

Advantageously, this defines the distance between the task definitions by normalizing each of the parameters of the task definition, such as the type of task to be performed between 0 and 1, so that all tasks are evaluated equally. Each type of task that may be performed can be represented by a set of parameters coefficient indicating the type of task it is, for example, the type of cognitive processes it involves. For each of the task definitions parameters, the distance between two task definition parameters can be computed as above, by computing the sum of the differences for all the task definition parameters and then normalizing it through the normalizing factor D_{N}. The normalizing factor D_{N} allows for normalizing the set of possible values d_{TDef} may take, such as the maximum difference between the task definition of two cases is 1, and the rest of the differences between the task definition of other cases are scaled according to such difference. This ensures the distance between the task definitions is always comprehended between 0 and 1, wherein 0 means that the tasks definitions are the same and 1 that the task definitions are the most different between any two cases. In a further preferred embodiment, the normalizing factor D_{N} is computed as: ${D}_{N} = max \left(\left|{TDef}_{i}\right|\left|{TDef}_{N}\right|\right)$

In another further preferred embodiment, the distance between the cognitive profiles of the users of cases i and N computed in step 204 is computed as: ${d}_{Prof \left(i, N\right)} = \frac{1}{{D}_{N}} {\sum }_{∀ cp} \left|{cpd}_{{u}_{i}}-{cpd}_{{u}_{N}}\right|$ *cpd_{uᵢ}* and *cpd_{u_{N}}* are the normalized coefficients ∈ [0,1] indicating the degree of impairment of the cognitive process cp for the users of cases i and N, respectively and D_{N} is a normalizing factor computed as the sum of max differences between the cpd values.

Advantageously, this defines the distance between the cognitive profiles of two users by normalizing each of the parameters of the cognitive profiles. A cognitive profile may be defined as a set of cognitive processes cp, wherein the coefficient cpd can be established. Preferably, the cpd values are normalized between 0 and 1 such that a user with no impairment in a given cognitive process would have a value in the dimension of such cognitive process of 0 and a user with a 100% impairment in a given cognitive process would have a value in the dimension of such cognitive process of 1. The impairment of the user for each cognitive process may be determined by a professional as a degree in any scale, that may be then transformed to a 0 to 1 scale. For each of the cognitive process coefficients cpd, the distance between two cognitive process impairment degrees parameters cpd can be computed as above, by computing the sum of the differences for all the cognitive process coefficients and then normalizing it through the normalizing factor D_{N}.

The normalizing factor D_{N}, allows for normalizing the set of possible values d_{Prof} may take, such as the maximum difference between the cognitive processes of two cases is 1, and the rest of the differences between the cognitive processes of other cases are scaled according to such difference. This ensures the distance between the cognitive profiles is always comprehended between 0 and 1, wherein 0 means that the cognitive profiles are the same and 1 that the cognitive profiles are the most different between any two cases. In a further preferred embodiment, the normalizing factor D_{N} is computed as: ${D}_{N} = max \left(\left|{cpd}_{i}\right|\left|{cpd}_{N}\right|\right)$

An alternative way of computing the cognitive distance may be, that for example given a cognitive profile defined by the user's sustained attention, selective attention and divided attention impairment, wherein the user of interest has a sustained attention impairment of x, a selective attention impairment of y and a divided attention impairment of z, the user cognitive profile can be represented as the vector [x, y, z] Given two such vectors, the distance between them may be computed as the subtraction of one vector from the other, then computing the module of the resulting vector and then normalizing it through the normalizing factor D_{N}.

In another further preferred embodiment, the distance between the task configurations of cases i and N computed in step 204 is computed as: ${d}_{Conf \left(i, N\right)} = \frac{1}{{D}_{N}} {\sum }_{∀ Conf} \left|{Conf}_{i}-{Conf}_{N}\right|$

*Confᵢ* and *Conf_{N}* are the normalized coefficients ∈ [0,1] indicating the task configuration parameter in each case i and N, respectively and D_{N} is a normalizing factor computed as the sum of max differences between the Conf values.

Advantageously, this defines the distance between the task configurations by normalizing each of the parameters of the tasks configurations. A task configuration may be defined as a set of parameters such as the length of the task, exposure time, interstimulus time, n-back, modality, size, sessions, trials, % of distractors, % of targets, delay, signal, stop, change and/or type of change. For a given task, a coefficient between 0 and 1 may be assigned to each of these parameters, wherein 0 may define, for example the shortest value or easiest configuration, and 1 the largest value or hardest configuration. It is noted that in some other embodiments, the values may be assigned differently. As long as the assignation is consistent across the cases, the distance will properly serve to determine the similarity between the tasks configurations.

For each of the task configuration parameters, the distance between two task configuration parameters can be computed as above, by computing the sum of the differences for all the task configuration parameters and then normalizing it through the normalizing factor D_{N} The normalizing factor D_{N} allows for normalizing the set of possible values d_{TDef} may take, such as the maximum difference between the task configuration of two cases is 1, and the rest of the differences between the task configuration of other cases are scaled according to such difference. This ensures the distance between the task configuration is always comprehended between 0 and 1, wherein 0 means that the tasks configurations are the same and 1 that the task configuration are the most different between any two cases. In a further preferred embodiment, the normalizing factor D_{N} is computed as: ${D}_{N} = max \left(\left|{Conf}_{i}\right|\left|{Conf}_{N}\right|\right)$

In another further preferred embodiment, the distance between the results of cases i and N computed in step 204 is computed as: ${d}_{Res \left(i, N\right)} = \frac{1}{{D}_{N}} {\sum }_{∀ Res} \left|{Res}_{i}-{Res}_{N}\right|$

*Resᵢ* and *Res_{N}* are the normalized coefficients ∈ [0,1] indicating the task result parameter in each case i and N, respectively and D_{N} is a normalizing factor computed as the sum of max differences between the Res values.

Advantageously, this defines the distance between the results of the cases by normalizing each of the parameters of the results. A task configuration may be defined as a set of parameters such as the success rate and/or fail rate. For a given task, a coefficient between 0 and 1 may be assigned to each of these parameters, wherein 0 may define, for example as a 0% rate, and 1 as a 100% rate.

For each of the result parameters, the distance between two result parameters can be computed as above, by computing the sum of the differences for all the result parameters and then normalizing it through the normalizing factor D_{N} The normalizing factor D_{N} allows for normalizing the set of possible values d_{Res} may take, such as the maximum difference between the results of two cases is 1, and the rest of the differences between the task configuration of other cases are scaled according to such difference. This ensures the distance between the results is always comprehended between 0 and 1, wherein 0 means that the results are the same and 1 that the task definitions are the most different between any two cases. In a further preferred embodiment, the normalizing factor D_{N} is computed as: ${D}_{N} = max \left(\left|{Res}_{i}\right|\left|{Res}_{N}\right|\right)$

In some embodiments of any of the previous embodiments of the first aspect of the invention, it may happen that the user has never performed a cognitive process rehabilitation task at all, or with a specific parameter task definition of parameter. In such cases, as there is no information relative to the task, nor to the results for the new proposed case it may not be intuitive how to initialize such parameters, given that we have no information about the user's performance.

Preferably in such embodiments and as shown with respect to Fig. 2, the method to determine the closest k cases, involves a step 203, of determining if there is previous information about the user to select the closest k cases to the user's new case by using a case-based reasoning (CBR) paradigm which comprises computing the distance between each case cᵢ of the set of past cases and the new case c_{N,,} This would be noted because a new case c_{N} cannot be defined as there is no information for some parameters of the task nor the result.

If there is enough information, then the distance of each past case with the new case is computed according to step 204. Otherwise, in step 254 the k cases are selected using a case-based reasoning (CBR) paradigm which comprises computing the distance of the cognitive profile of the user of each past case i from the set of past cases with the new user N wherein the users of the past cases have good rehabilitation results, which is computed as: ${d}_{\left({u}_{i}, {u}_{N}\right)} = \frac{1}{{D}_{N}} {\sum }_{∀ cp} \left|{cpd}_{{u}_{i}}-{cpd}_{{u}_{N}}\right|$

*cpd_{uᵢ}* and *cpd_{u_{N}}* are the normalized coefficients ∈ [0,1] indicating the degree of impairment of the cognitive process cp for the users of cases i and N, respectively and D_{N} is a normalizing factor computed as the sum of max differences between the cpd values.

Advantageously, this defines the distance between the cognitive profiles of each of the users of past cases which have good rehabilitation results with the current user such that the distance of each of the parameters of the cognitive profiles is normalized.

A cognitive profile may be defined as a set of cognitive processes cp, wherein the coefficient cpd can be established. Preferably, the cpd values are normalized between 0 and 1 such that a user with no impairment in a given cognitive process would have a value in the dimension of such cognitive process of 0 and a user with a 100% impairment in a given cognitive process would have a value in the dimension of such cognitive process of 1. The impairment of the user for each cognitive process may be determined by a professional as a degree in any scale, that may be then transformed to a 0 to 1 scale. For each of the cognitive process coefficients cpd, the distance between two cognitive process impairment degrees parameters cpd can be computed as above, by computing the sum of the differences for all the cognitive process coefficients and then normalizing it through the normalizing factor D_{N}. The normalizing factor D_{N}, allows for normalizing the set of possible values d_{Prof} may take, such as the maximum difference between the cognitive processes of two cases is 1, and the rest of the differences between the cognitive processes of other cases are scaled according to such difference. This ensures the distance between the cognitive profiles is always comprehended between 0 and 1, wherein 0 means that the cognitive profiles are the same and 1 that the cognitive profiles are the most different between any two cases. In a further preferred embodiment, the normalizing factor D_{N} is computed as: ${D}_{N} = max \left(\left|{cpd}_{i}\right|\left|{cpd}_{N}\right|\right)$

Advantageously, determining the distance between the cognitive profiles of each of the users of past cases with the current user allows to determine a set of k past cases which define a good subset of past users' cases that, for such user, represent parameters that lead to a good rehabilitation of the user of interest.

Then, in step 256, the similarity of each case with the new case is computed, based on the distance of each past case with the new case computed in step 254. The similarity may be simply computed as an inverse function of the distance or may comprise a more complex function that considers more information of each case to determine the similarity of each past case with the new case. This may comprise for example a machine learning or artificial intelligence algorithm that uses the distance as an input to compute the similarity. It is noted that to determine the similarity other parameters apart from the distance between the cognitive profiles of each of the users of past cases with the current user may be used, to discriminate between the different cases with good rehabilitation results of each user.

Then, once the similarity of each past case with the new case is computed, in the step 208 the set of k closest past cases may be defined as the set of k cases with the most similarity with the new case. In some embodiment, it may be required that some diversity is found across the set of the selected k cases, for example by requiring that the set of k past cases comprise at most m number of cases from the same user, wherein m< k. Advantageously, this prevents the overfitting to a certain subject. In some embodiments, m = 1, this is, only a case per user is allowed.

Finally, the set of k closest cases are provided to the computer-implemented method for determining the optimal parameters for the progression of a cognitive process rehabilitation task of a user in step 104. In some embodiments, the set of k cases provided onto step 104 may be limited according to the requirements of the method, for example, by selecting a subset of the set of k cases. It is noted that the first time of a user, only the initial values of the parameters of each selected case may be averaged in subsequent step 106.

According to another preferred embodiment, when there are there are not k cases in the set of past cases, either because k is large or because there are still too little cases in the set of past cases, instead of selecting a set of k cases to determine the optimal parameters, the parameter values are a set of initial predetermined values that provide a reasonably good starting point.

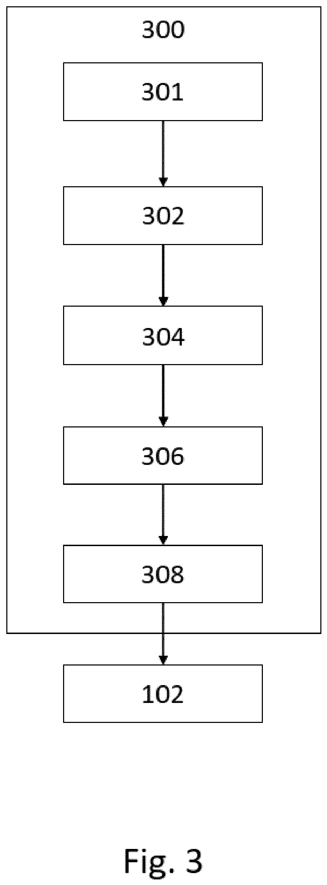

In another preferred embodiment, as shown with respect to Figs.1 and 3, the task to be performed by the user is determined by a computer-implemented method 300 that comprises several steps. First, as shown in step 301, it comprises receiving a set of cognitive rehabilitation tasks. These tasks may be any type of tasks either computer-implemented or physical that can serve as a cognitive process rehabilitation task for a user with some degree of cognitive impairment. Therefore, they are tasks that involve exercising some type of cognitive process such as processing speed, decision making, inhibitory control or cognitive flexibility. The tasks comprise one or more modifiable parameters, selected from: length of the task, exposure time, interstimulus time, n-back, modality, size, sessions, trials, % of distractors, % of targets, delay, signal, stop, change or type of change. This, allow for the tasks to be suitable to be tuned by the progression parameter optimization computer-implemented method in order to provide an adaptative and personalised progression during the rehabilitation to the user.

Then, in step 301 the method comprises determining the suitability of each task of step 301 to the user's cognitive profile. This may be done according to the relationship between the cognitive profile of the user and the cognitive process each task exercises. More preferably, the suitability of the task comprises computing the distance between the task t and the user's cognitive profile u, computed as: ${d}_{\left(u, t\right)} = \frac{1}{D} {\sum }_{cp ∈ {u}_{cp}} cpd \left(maxPr-{Pr}_{{t}_{cp}}\right)$
wherein cp is a cognitive process, cpd is the normalized coefficient ∈ [0,1] indicating the degree of impairment of the cognitive process cp, *Pr_{t_{cp}}* is the priority of the task t for such cognitive process cp, maxPr is a stablished maximum priority value, and D is a distance normalizing factor, computed as: $D = max \left(\left|{u}_{cp}\right|\left|{t}_{cp}\right|\right) maxPr$
wherein u_{cp} is the set of cognitive processes impaired in the user u, and t_{cp} is the set of cognitive processes related with task t.

Advantageously, this defines a distance between the cognitive profiles of the user and each of the tasks from the set of cognitive rehabilitation tasks such that a parameter of comparison between tasks can be stablished. Then the normalization parameter D allows for normalizing the set of possible values d(u,t) may take, such as the maximum difference between the a given task and the cognitive profile of the user is 1, and the rest of the differences between the rest of the tasks and the cognitive profile of the user are scaled according to such difference. This ensures the distance between the tasks and the cognitive profile of the user is always comprehended between 0 and 1, wherein 0 means that the task and the cognitive profiles of the user fit equally, i.e. that the task fits perfectly to train the cognitive impairments of the user; and 1 that the task and the cognitive profile of the user are the most different between any two cases.

Once the distance d(u, t) has been computed, the suitability of each task is computed, based on the distance of each task with the cognitive profile of the used. The suitability may be simply computed as an inverse function of the distance d(u, t) or may comprise a more complex function that considers more information of each task to determine the suitability of each task with the user. This may comprise for example a machine learning or artificial intelligence algorithm that uses the distance as an input to compute the suitability.

Next, in step 304 the method comprises sorting the set of tasks of step 301 according to the computed suitability determined in step 302. This is preferably done from in decreasing order, such that those tasks with higher suitability are the first elements of the list. However, it may be noted that the set of tasks may be ordered in alternative ways according to the computed suitability such as in a decreasing order and still provide a useful sorted set of tasks.

Then in step 306, the method comprises providing the sorted set of tasks of step 304 or a selected subset of the most suitable tasks of the sorted set of tasks of step b) to a therapist. Although there a suitability rank may be provided, the computer-implemented method cannot perform a treatment decision, and therefore a therapist shall take the decision of which tasks among all the tasks or a selected subset of the most suitable tasks is really the most suitable for the user.

Finally, in step 308, the method comprises receiving the task to be performed from the therapist, wherein the task is a task from the set or subset of tasks provided in step 306 to the therapist. Therefore the method successfully achieves to retrieve a task from the received set of cognitive rehabilitation tasks of step 301 which is the most suitable for the user, and that therefore shall be performed by the user.

Once the task to be performed the user is determined, it is provided to the computer-implemented method for determining the optimal parameters for the progression of a cognitive process rehabilitation task of a user in step 102.

In another preferred embodiment, the values of the modifiable parameters of each case are averaged by weight averaging them according to the result of the task. Advantageously, this allows for the method to take into account the result of each case when determining the modifiable parameters of a new case. More preferably the weight averaging comprises increasing the weight for those cases with an excessively good result, and reducing the weight for those cases with a bad result. Thus, if a certain set of parameters lead to good rehabilitation result, it will have a greater effect on the selected optimal modifiable parameter, while if a certain set of parameters lead to bad rehabilitation result, it will have a smaller effect on the selected optimal modifiable parameter.

In a further preferred embodiment, the result of the task is considered excessively good when the success rate is equal or higher than 95% and the fail rate is lower than 5%. Alternatively or additionally, the result of the task is considered bad when the success rate is lower than 40% and the fail rate is higher than 30%. In some embodiments, wherein the task is not considered excessively good or bad according to the aforementioned threshold, the result of the task is considered good.

Moreover, in some embodiments the determination of the result of the task may further serve as an input to an Al or machine learning to determine the optimal parameters. Therefore, the update of the optimal parameters may be weighted by how good a result of a task has been, for example, a task with a 60% of success rate will increase the modifiable parameters more than a task with a 40% of success rate, as the former shows a better progression than the latter. In other embodiments, when the result has extreme values, such as when the success rate is extremely high or low and/or the fail rate is extremely high or low, the Al or machine learning algorithm may determine that the parameters were set too difficult or too low, reacting to ease or harden the difficulty by updating the parameters, respectively.

According to another preferred embodiment, the cognitive process is one or more cognitive processes selected from: sustained attention, selective attention, divided attention, processing speed, decision making, inhibitory control, cognitive flexibility and/or operative memory. It is noted, that in some embodiments the cognitive processes rehabilitated may comprise other cognitive processes not described herein whose rehabilitation progress through a task may be optimized with any of the methods according to the first aspect of the invention, as the skilled person may note.

In another preferred embodiment, the CBR paradigm comprises taking into account the age of the users. Advantageously, this allows for a selection of the k closest cases that take into account the effect of the age on the rehabilitation progress optimization. This in turn allows to select the tasks and parameters taking into account the effect of the age. For example, a case from a 5-year-old child may not be as relevant for a 72-year-old woman as a case from a 76-year-old man. Therefore, the CBR paradigm implementation, for example, through an Al or machine learning algorithm, may penalize the 5-year-old child case compared to the 76-year-old man when selecting the closest k cases for the 72-year-old woman rehabilitation progress optimisation. This further makes the cognitive rehabilitation progress optimisation method even more personalised.

According to another preferred embodiment, the CBR paradigm comprises taking into account the diagnostic of the users. Some users may be diagnosed with some mental condition such as ADHD, dementia or Alzheimer's disease, among other. These mental conditions may lead to similar cognitive profiles in some cases, while the rehabilitation requirements may not be the same. Therefore, advantageously, this allows the CBR paradigm to be able to consider the diagnostic of the user, making the cognitive rehabilitation progress optimisation method even more personalised.

In another preferred embodiment, the task is selected and/or the modifiable parameters are determined taking into account diverse conditions of the subject that may be a handicap for the user. For example, for a deaf user, the system may consider discarding any cognitive task that comprise relevant sounds as part of the interaction. If a user has motor slowness, the modifiable interstimulus time parameter shall not change, as it is a function the user cannot evolve, and if modified, the progression of the cognitive process rehabilitation may be negatively affected. As another exemplary embodiment, if a user suffers from bradykinesia, the tasks shall be adapted to the user's intervals of interacting, by adapting towards longer intervals for all type of time-related modifiable parameters. Another example may be the case of a user with hemispatial neglect, wherein the tasks shall be adapted such that the stimuli are presented on the area wherein the perception is preserved. Advantageously, this further allows for the cognitive rehabilitation progress optimisation method to be even more personalised. In a more preferred embodiment, the conditions comprise one or more of: deafness, motor slowness, bradykinesia or hemispatial neglect.

A second aspect of the invention, refers to a computer program product comprising instructions such that when processed by a processor, configures it to perform any of the methods according to any of the embodiments of the first aspect of the invention.

Figure 4 shows a computer program product 400 to implement the methods according to any of the embodiments of the first aspect of the invention. The product may be saved in a memory unit such as one or more volatile or non-volatile memory devices, such as DRAM, SRAM, flash memory, read-only memory, ferroelectric RAM, hard disk drives, floppy disks, magnetic tapes, optical disks or the like. It is noted that the processor may comprise one or more processing units, such as a microprocessor, GPU, CPU, multi-core processor or the like. The program and its modules may be stored in a local device memory (e.g. in the device executing the task), or it can be stored remotely, e.g. on a remote server connected communicatively to the device. Similarly, the program and its modules may be processed by a local processor (e.g. in the device executing the task), or it can be processed remotely, e.g., on a remote server connected communicatively to the device.

The computer program product 400 comprises an averaging module 410 configured to average a set of k cases from a set of past cases, wherein the set of k cases have been selected according to a CBR paradigm. The averaging module is configured to receive the set of k cases and the task to be performed by the user with the set of modifiable parameters comprises in the task, and provide an average of the modifiable parameters of the set of k cases, as described with respect to the step 106 in the first aspect of the invention. It is noted the average may be performed according to any of the embodiments of the first aspect of the invention, including the weighting averaging.

The computer program product 400 further comprises a determination module 420, configured to receive from the averaging module 410 the average of the modifiable parameters of the set of k cases and set the modifiable parameters of the current case, to be such parameters, as described with respect to the step 108 in the first aspect of the invention. The determination module 420 may be configured to send these set of parameters to the module of the device configuring the task parameters.

The computer program product 400 also comprises an updating module 430, configured to include to the set of past cases the current case once the task is finished, comprising the case task definition, user's cognitive profile, task configuration and task results, as described with respect to the step 110 in the first aspect of the invention.

In some embodiments, the computer program product 400 may further comprise a task selection module 440. The task selection module 440 is configured to receive a set of possible tasks and provide the task to be performed by the user. The task selection module comprises a suitability module 442, configured to determine the suitability of the task to the user's cognitive profile, as described with respect to the step 302 in the first aspect of the invention. The task selection module also comprises a sorting module 444, configured to sort the set of tasks received according to the suitability of the task to the user's cognitive profile determined by the suitability module 442, as described with respect to the step 304 in the first aspect of the invention. The task selection module also comprises a interaction module 446, configured to provide the set of sorted tasks of the sorting module 444 to or a selected subset of the most suitable tasks of the sorted set of tasks of the sorting module 444 to a therapist, and receiving from the therapist the task to be performed from the set or subset of tasks provided, as described with respect to the steps 306 and 308 in the first aspect of the invention.

In some embodiments, the computer program product 400 may further comprise a case set selection module 450. The case set selection module 450 is configured to receive a set of past cases and provide a set of k cases, wherein the k cases are the closest k cases to the user's new case, as described with respect to any of the method 200 of the first aspect of the invention. The case set selection module 450 comprises a distance computing module 454, configured to determine the distance between each of the past cases and the new proposed case as described with respect to the steps 204 and 254 in the first aspect of the invention. The case set selection module 450 also comprises a similarity computing module 456, wherein the similarity between each of the past cases and the new proposed case is computed from the distance computed in the distance computing module 454, as described with respect to the steps 206 and 256 in the first aspect of the invention. Finally, the case set selection module 450 comprises a k-selection module 458, wherein the set of k closest past cases is defined as the set of k cases with the most similarity with the new case as computed by the similarity computing module 456, as described with respect to the step 208 in the first aspect of the invention.

All of the above are fully within the scope of the present disclosure, and are considered to form the basis for alternative embodiments in which one or more combinations of the above described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

### EXAMPLES

### Example 1: Predictive value of the method

### Materials and methods

The objective of this first study was to calculate the predictive value of patient improvement in the cognitive activities of the proposed method and its relationship with the Conners Continuous Performance Test (CPT-III), a computerised standardised continuous performance test used in the assessment of ADHD. Finally, the predictive value of relationship with the clinical measure Scale for the Evaluation of Attention Deficit Hyperactivity Disorder (EDAH) was calculated. EDAH consists of 20-items and aims to assess the main ADHD features and any coexisting behavioural disorders. EDAH includes four index scores: Inattention (EDAH_DA), hyperactivity (EDAH_H), hyperactivity and inattention (EDAH_DAH) behavioural disorders (EDAH_TC) and the global indexes.
A) Method:
   - Sample selection:
      ∘ Minimum 15 workouts per game. 15 workouts represent a positive change in performances of those patients playing more than 15 workouts relative to those playing less than 15 workouts.
   - Target generation
      ∘ Difference of performance of the last 25th percentile sessions and performance of the first training.
      ∘ If the difference is greater than the determined threshold (sigma) there is improvement.
   - Feature engineering
      ∘ Parameters: duration, difficulty, hit rate
      ∘ Statistics: 25th, 50th, 75th quartiles

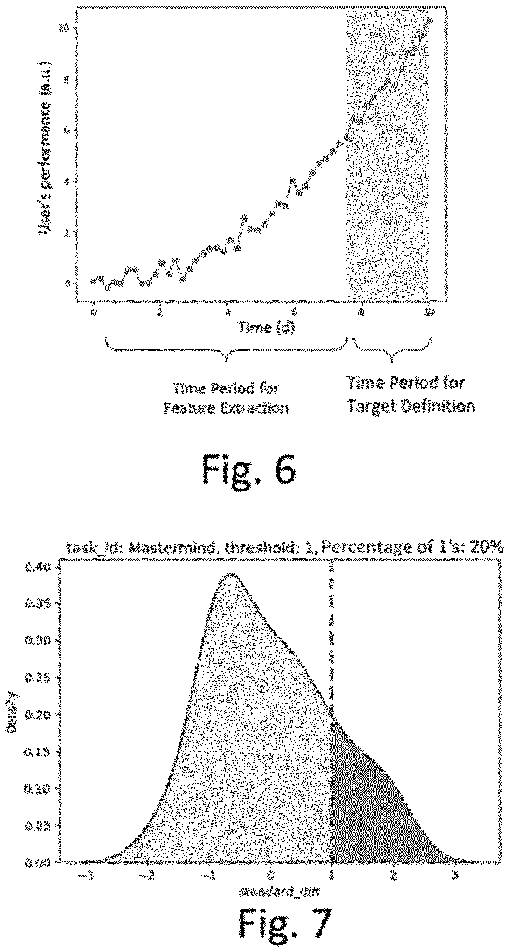

Figure 6 graphically illustrates the time intervals selected for the modelling process in accordance with one or various embodiments of the invention. On the horizontal axis, the 'Time Period for Feature Extraction' is highlighted, referring to the span during which data is gathered and processed to be used as features in the model; whereas the shaded region denotes the 'Time Period for Target Definition', which specifies the interval wherein the goal the model aims to predict is established or defined.

Figure 7 shows the distribution of the difference in performance of the last quartile vs. the baseline. The vertical line is the 1 standard deviation cut-off for improvement.

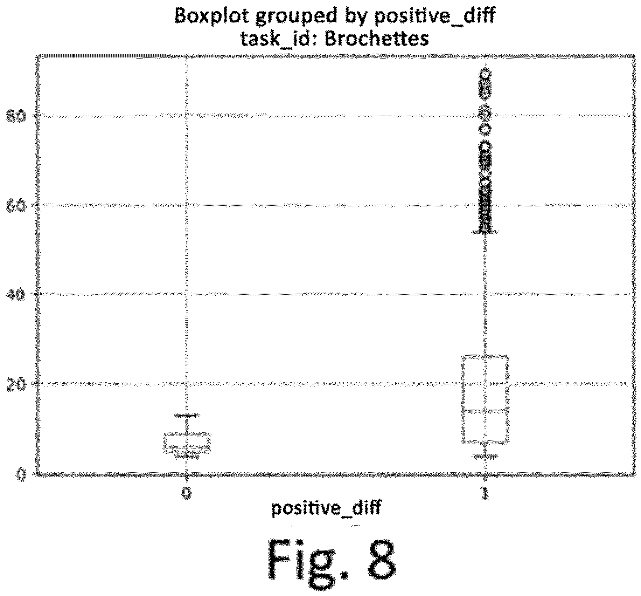

Figure 8 shows the distribution of number of training sessions per patient vs. improvement (0 no improvement, 1 improvement). The patient must play each cognitive task at least 15 times. This graph shows that on average there is an improvement in performance from training 15 onwards.

### B) General statistics

| Task | N | M (SD) treatment sessions | Age | Target percentage | Percentage males | Percentage females |
|---|---|---|---|---|---|---|
| Poker | 364 | 19 ± 3,7 | 11 ± 1,3 | 11,54% | 69,78% | 30,22% |
| Racer | 315 | 30 ± 13,3 | 10 ± 1,3 | 20,00% | 73,02% | 26,98% |
| Sequence | 284 | 36 ± 24,6 | 11 ± 1,3 | 14,79% | 72,18% | 27,82% |
| Labyrinth | 377 | 35 ± 18,2 | 10 ± 1,3 | 20,69% | 70,82% | 29,18% |
| Mastermind | 79 | 31 ± 13,6 | 11 ± 1,2 | 20,25% | 64,56% | 35,44% |
| Bosque | 336 | 26 ± 7,4 | 10 ± 1,3 | 16,67% | 72,62% | 27,38% |
| Selection | 305 | 35 ± 17,6 | 11 ± 1,3 | 20,33% | 73,44% | 26,56% |
| Brochettes | 351 | 33 ± 17,3 | 10 ± 1,3 | 21,94% | 71,23% | 28,77% |
| Invasion | 308 | 32 ± 15,4 | 10 ± 1,3 | 21,75% | 71,43% | 28,57% |
| Memory | 314 | 32 ± 15 | 11 ± 1,3 | 19,11% | 71,66% | 28,34% |
| Gong | 326 | 39 ± 29,4 | 11 ± 1,3 | 14,72% | 72,70% | 27,30% |
| Capture | 382 | 29 ± 10,7 | 10 ± 1,3 | 16,23% | 71,20% | 28,80% |
| Cancellation | 292 | 30 ± 12,9 | 11 ± 1,3 | 12,67% | 73,29% | 26,71% |
| Tetris | 94 | 28 ± 10,7 | 11 ± 0,8 | 17,02% | 64,89% | 35,11% |

- Statistics per game:
   ∘ Number of cases: number of patients, fulfilling the conditions, used for training Machine Learning models.
   ∘ Training: average number of trainings +- standard deviation.
   ∘ Age: average age +-standard deviation.
   ∘ % target: percentage of patients who have improved (using the formula discussed in the previous slide).
   ∘ % of men (boys): percentage of patients who are men.
   ∘ % female (girls): percentage of patients who are female.

### C) Models:

- Models tested:
   ∘ Logistic regression (1): assumes linear dependence between input and output variables.
   ∘ Random Forest (2): combines multiple classification trees. Each tree learns independently. It is more robust and with better accuracy than a single classification tree.
   ∘ Gradient Boosting (3): combines multiple classification trees. Each tree learns sequentially. Each tree learns from the errors of previous trees.
- Validation criterion:
   ∘ AUC
- Validation technique:
   ∘ Cross-validation.
- Best performing model: Random Forest

### Results

### Machine learning results on real patient data

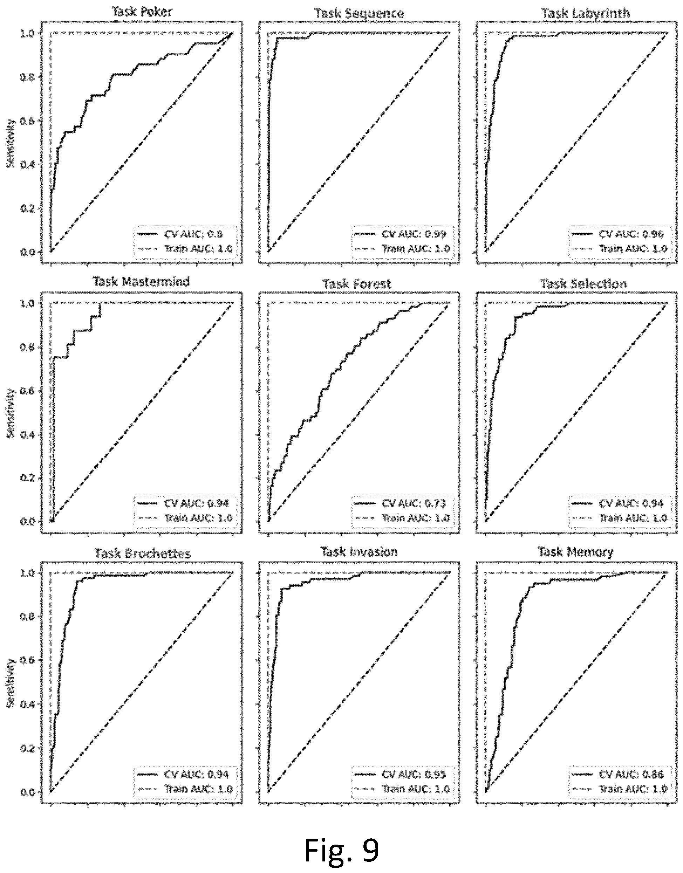

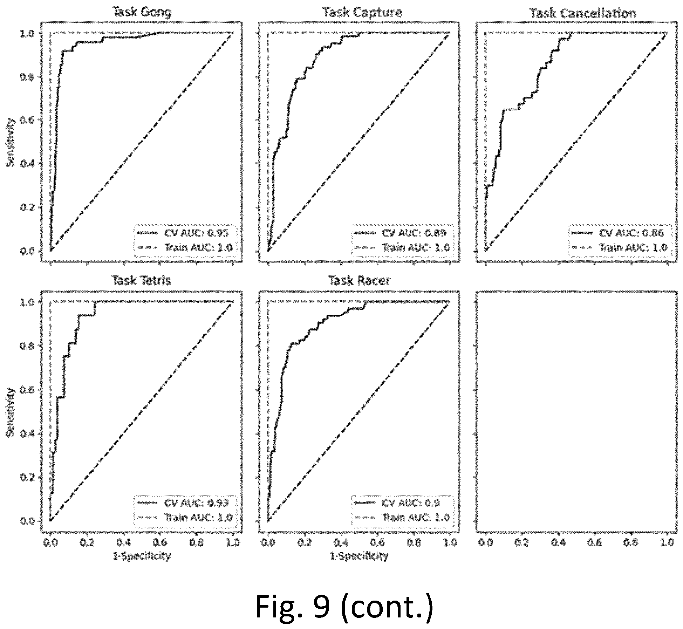

Figure 9 shows machine learning results on real patient data, where it can be appreciated the improvement accuracy of patients in different cognitive tasks. The dashed line shows the ROC and AUC in the training set and the solid line the ROC and AUC in the test set using cross-validation.

### Machine learning results on CPT-III

### CPT-III Commissions

Figs, 10 and 11 shows the ROC curve related to commissions and the most important features per cognitive task in the construction of the predictive model in percentage, respectively.

### CPT- III Perseverations

Figs, 12 and 13 shows the ROC curve related to perseverations and the most important features per cognitive task in the construction of the predictive model in percentage, respectively.

### CPT-III Omissions

Figs, 14 and 15 shows the ROC curve related to omissions and the most important features per cognitive task in the construction of the predictive model in percentage, respectively.

### CPT-III Variability

Figs, 16 and 17 shows the ROC curve related to response variability and the most important features per cognitive task in the construction of the predictive model in percentage, respectively.

### Machine learning results on the clinical measure EDAH

### EDAH- Attention Deficit (AD)

Figs, 18 and 19 shows the ROC curve related to attention deficit and the most important features per cognitive task in the construction of the predictive model in percentage, respectively.

### EDAH- Hyperactivity (H)

Figs, 20 and 21 shows the ROC curve related to hyperactivity and the most important features per cognitive task in the construction of the predictive model in percentage, respectively.

### EDAH- Attention Deficit (AD) and hyperactivity (H)

Figs, 22 and 23 shows the ROC curve related to attention deficit and hyperactivity and the most important features per cognitive task in the construction of the predictive model in percentage, respectively.

### Conclusion

Predictive models with real patient data have shown that both cognitive tasks is effective in generating improvement in patients. At the same time, these same data demonstrated a robust relationship with traditional measures such as the CPT-III and EDAH.

### Example 2: Comparative Evaluation of Difficulty Generation in Cognitive Games Using Different Distance Estimates

The objective of this first study was to compare two approaches to generate levels of difficulty in cognitive tasks through our Al: using two distances (profiles and games) and four distances (profiles, games, settings and results).

The application of Artificial Intelligence (AI) in cognitive tasks seeks to adapt, personalise and improve the patient's cognitive process. Proper difficulty tuning is crucial to ensure an appropriate challenge for each patient. This research compares two tuning methods to determine which provides a more personalised experience.

Case-Based Reasoning (CBR) is an Al approach where a database of previous cases is searched to find similar solutions to a new problem. The term "distance" is a measure used in CBR to determine how similar a new case is to previous cases.

Evaluation: A metric that reflects the patient's performance and progression during a specific session of a cognitive task. Evaluation is used to determine the effectiveness of the current environment and to inform the Al about adaptations needed for future sessions. It is a value between 0 and 1 derived from the results. The progress is measured as a value between 0 and 1 derived from the game settings.

### Materials and methods

The Al employs the CBR approach. It starts by searching for K similar cases using a sum of 4 distances, task configuration, profile, configuration and result: ${d}_{\left({c}_{i}, {c}_{N}\right)} = {d}_{{TDef}_{i , N}} + {d}_{{Prof}_{i , N}} + {d}_{{Conf}_{i , N}} + {d}_{{Res}_{i , N}}$

If the patient is playing for the first time, two distances are used, cognitive task and profile: ${d}_{\left({c}_{i}, {c}_{N}\right)} = {d}_{{TDef}_{i , N}} + {d}_{{Prof}_{i , N}}$

The configuration generated in the next game is derived from these close cases.

The "evaluation" metric has been chosen as the main comparison tool for its ability to directly reflect the adaptability and effectiveness of the difficulty generated by the Al. An adequate evaluation indicates that the level of difficulty is neither too easy nor too difficult for the patient, suggesting personalised adaptation. Comparison between the 2-distance and 4-distance approaches using this metric offers direct insight into which method results in a more optimised patient experience.

### Results

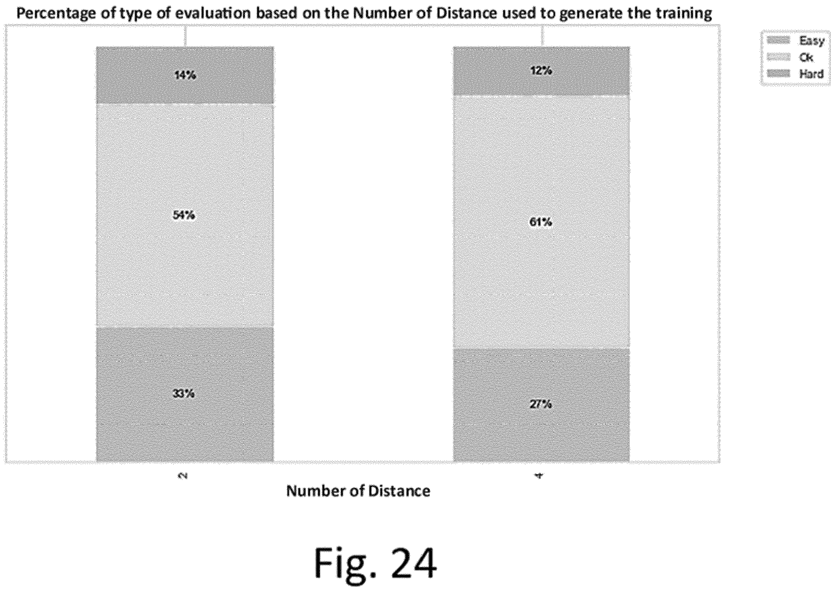

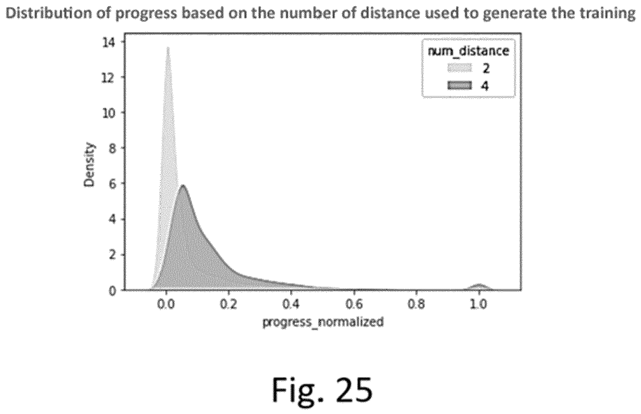

Fig. 24 shows a graphical representation comparing the percentage of "evaluation" as a function of the number of distances used. This graph illustrates the differences in the "Easy", "Ok", and "Hard" categories when using the 2-distance approach (on the left) versus the 4-distance approach (on the right). A noticeable increase is observed in the "Ok" category from 54% to 61% when switching from 2 to 4 distances, while the "Easy" and "Hard" categories show reductions when using the 4-distance approach. This visualisation reinforces the idea that the use of 4 distances provides a more balanced and personalised experience for the player.
A) Difficulty assessment:
   The exact numerical proportion of assessments categorised as "Ok" was 0.536511 with 2 distances and 0.606587 with 4 distances.
B) Statistical Analysis
   Statistical analyses were carried out using Python 3.10 software. Two proportion test were conducted with an α=0.05 resulting in a z-value of -32.49 and a p-value of 0.0000 were obtained, indicating a statistically significant difference between the two approaches.
C) Visual Comparison of Progress
   A visualisation of the distribution of progress between the two distance methods is shown in Fig. 25. The graph reveals a clear differentiation between the two methodologies. It can be seen that when using 2 distances, progress tends to be concentrated in lower values, indicating easier configurations. On the other hand, when using 4 distances, the distribution is more dispersed and leans towards higher difficulty settings, reflecting a more personalised adjustment to the player's profile and experience.

### Conclusions

The increase in the proportion of "Ok" with 4 distances underlines the relevance of including previous settings and results. The progress analysis also suggests a more challenging and personalised adaptation with the 4-distance approach.

The 4-distance approach to cognitive game difficulty adjustment is significantly superior in terms of customisation and adaptability, as indicated by the "evaluation" metric and statistical analysis.

## Claims

1. A computer-implemented method for determining the optimal parameters for the progression of a cognitive process rehabilitation task of a user, wherein the progression is adaptative and personalised, comprising:
a. receiving a determined task to be performed by the user, the task comprising modifiable parameters affecting the difficulty of the task;
b. receiving a set of k cases from a set of past cases, wherein the k cases are selected using a case-based reasoning (CBR) paradigm;
c. averaging the values of the modifiable parameters of the set of k cases received in step b);
d. determining the optimal parameters of the task as the averaged modifiable parameters of step c); and
e. updating the set of past cases with the results of the task performed with the optimal parameters of step d);
wherein the modifiable parameters comprise one or more parameters selected from: length of the task, exposure time, interstimulus time, n-back, modality, sessions, trials, % of distractors, % of targets, delay, signal, stop or type of change,
and wherein the received k cases of step b) are the closest k cases to the user's new case and are selected using the CBR paradigm which comprises computing the distance between each case cᵢ of the set of past cases and the new case c_{N}, the distance computed as: ${d}_{\left({c}_{i}, {c}_{N}\right)} = {d}_{{TDef}_{i , N}} + {d}_{{Prof}_{i , N}} + {d}_{{Conf}_{i , N}} + {d}_{{Res}_{i , N}}$
wherein *d_{TDef_{i,N}}* is the distance between the task definitions of cases i and N, *d_{Prof_{i,N}}* is the distance between the cognitive profiles of users of cases i and N, *d_{Conf_{í,N}}* is the distance between the tasks configurations of the cases i and N, and *d_{Res_{í,N}}* is the distance between the results of cases i and N, and
wherein the distance between task definitions, cognitive profiles, task configurations and results is defined as an arbitrary value defining the difference between each two task definitions, cognitive profiles, task configurations and results, respectively, wherein one or more parameters of each task definition, cognitive profile, task configuration or result is used to compute such distance, respectively.

2. The computer-implemented method according to claim 1, wherein the received k cases are the closest k cases to the user's new case and are selected using the case-based reasoning (CBR) paradigm which comprises computing the distance between each case cᵢ of the set of past cases and the new case c_{N}, the distance between cases defined as an arbitrary value defining the difference between each two cases, wherein one or more parameters of each case is used to compute such distance.

3. The computer-implemented method according to any one of claims 1 or 2, wherein the distance between the task definitions of cases i and N is computed as ${d}_{TDef \left(i, N\right)} = \frac{1}{{D}_{N}} {\sum }_{∀ TDef} \left|{TDef}_{i}-{TDef}_{N}\right|$ wherein *TDefᵢ* and *TDef_{N}* are the normalized coefficients ∈ [0,1] indicating the task definition parameter in each case i and N, respectively and D_{N} is a normalizing factor computed as the sum of max differences between the TDef values.

4. The computer-implemented method according to any one of claims1 to 3, wherein the distance between the cognitive profiles of the users of cases i and N is computed as ${d}_{Prof \left(i, N\right)} = \frac{1}{{D}_{N}} {\sum }_{∀ cp} \left|{cpd}_{{u}_{i}}-{cpd}_{{u}_{N}}\right|$ wherein *cpd_{uᵢ}* and *cpd_{u_{N}}* are the normalized coefficients ∈ [0,1] indicating the degree of impairment of the cognitive process cp for the users of cases i and N, respectively and D_{N} is a normalizing factor computed as the sum of max differences between the cpd values.

5. The computer-implemented method according to any one of the claims 1 to 4, wherein the distance between the tasks configurations of cases i and N is computed as ${d}_{Conf \left(i, N\right)} = \frac{1}{{D}_{N}} {\sum }_{∀ Conf} \left|{Conf}_{i}-{Conf}_{N}\right|$ wherein *Confᵢ* and *Conf_{N}* are the normalized coefficients ∈ [0,1] indicating the task configuration parameter in each case i and N, respectively and D_{N} is a normalizing factor computed as the sum of max differences between the Conf values.

6. The computer-implemented method according to any one of the claims 1 to 5, wherein the distance between the results of cases i and N is computed as ${d}_{Res \left(i, N\right)} = \frac{1}{{D}_{N}} {\sum }_{∀ Res} \left|{Res}_{i}-{Res}_{N}\right|$ wherein *Resᵢ* and *Res_{N}* are the normalized coefficients ∈ [0,1] indicating the task result parameter in each case i and N, respectively and D_{N} is a normalizing factor computed as the sum of max differences between the Res values.

7. The computer-implemented method according to any one of the claims 1 to 6, wherein the first time for the user, the k cases are selected using the case-based reasoning (CBR) paradigm which comprises computing the distance of the cognitive profile of the user of each past case i from the set of past cases with the new user wherein the users of the past cases have good rehabilitation results as; ${d}_{\left({u}_{i}, {u}_{N}\right)} = \frac{1}{{D}_{N}} {\sum }_{∀ cp} \left|{cpd}_{{u}_{i}}-{cpd}_{{u}_{N}}\right|$ wherein *cpd_{uᵢ}* and *cpd_{u_{N}}* are the normalized coefficients ∈ [0,1] indicating the degree of impairment of the cognitive process cp for the users of cases i and N, respectively and D_{N} is a normalizing factor computed as the sum of max differences between the cpd values.

8. The computer-implemented method according to any one of the claims 1 to 7, wherein the task to be performed by the user is determined by:
a. receiving a set of cognitive rehabilitation tasks,
b. determining the suitability of each task of step a) to the user's cognitive profile,
c. sorting the set of tasks of step a) according to the suitability of the task to the user's cognitive profile of step b),
d. providing the sorted set of tasks of step c) or a selected subset of the most suitable tasks of the sorted set of tasks of step c) to a therapist,
e. receiving the task to be performed from the therapist, wherein the task is a task from the set or subset of tasks provided in step d) to the therapist.
wherein determining the suitability of the task comprises computing the distance between the task t and the user's cognitive profile u, computed as: ${d}_{\left(u, t\right)} = \frac{1}{D} {\sum }_{cp ∈ {u}_{cp}} cpd \left(maxPr-{Pr}_{{t}_{cp}}\right)$
wherein cp is a cognitive process, cpd is the normalized coefficient ∈ [0,1] indicating the degree of impairment of the cognitive process cp, *Pr_{tcp}* is the priority of the task t for such cognitive process cp, maxPr is a stablished maximum priority value, and D is a distance normalizing factor, computed as: $D = max \left(\left|{u}_{cp}\right|\left|{t}_{cp}\right|\right) maxPr$
wherein u_{cp} is the set of cognitive processes impaired in the user u, and t_{cp} is the set of cognitive processes related with task t.

9. The computer-implemented method according to any one of the claims 1 to 8, wherein the values of the modifiable parameters of each case are averaged by weight averaging according to the result of the task, increasing the weight for those cases with an excessively good result, and reducing the weight for those cases with a bad result.

10. The computer-implemented method according to any one of the claim 1 to 9, wherein the cognitive process is one or more cognitive process selected from: sustained attention, selective attention, divided attention, processing speed, decision making, inhibitory control, cognitive flexibility and/or operative memory.

11. The computer-implemented method according to any one of the claim 1 to 10, wherein the CBR paradigm comprises taking into account the age of the users.

12. The computer-implemented method according to any one of the claims 1 to 11, wherein the CBR paradigm comprises taking into account the diagnostic of the users.

13. The computer-implemented method according to any one of the claims 1 to 12, wherein the task is selected and/or the modifiable parameters are determined taking into account diverse conditions of the user that may be a handicap for the user,
preferably wherein the conditions comprise one or more of: deafness, motor slowness, bradykinesia or hemispatial neglect.

14. A computer program product comprising instructions such that when processed by a processor, configures it to perform any one of the methods according to claims 1 to 13.

## Patentansprüche

1. Computerimplementiertes Verfahren zur Bestimmung der optimalen Parameter für das Fortschreiten einer Rehabilitationsaufgabe für kognitive Prozesse eines Benutzers, wobei das Fortschreiten adaptiv und personalisiert ist, umfassend:
a. das Erhalten einer bestimmten Aufgabe, welche vom Benutzer durchgeführt werden soll, wobei die Aufgabe veränderbare Parameter umfasst, welche die Schwierigkeit der Aufgabe beeinflussen;
b. das Erhalten eines Satzes von k Fällen aus einem Satz von vergangenen Fällen, wobei die k Fälle unter Verwendung eines fallbasierten Schließen (CBR)-Paradigmas ausgewählt werden;
c. das Mitteln der Werte der veränderbaren Parameter des Satzes von k Fällen erhalten in Schritt b);
d. das Bestimmen der optimalen Parameter der Aufgabe als gemittelte veränderbare Parameter aus Schritt c); und
e. das Aktualisieren des Satzes von vergangenen Fällen mit den Ergebnissen der mit den optimalen Parametern aus Schritt d) durchgeführten Aufgabe;
wobei die veränderbaren Parameter einen oder mehrere Parameter umfassen, ausgewählt aus: der Dauer der Aufgabe, der Expositionszeit, der Interstimuluszeit, dem n-back, der Modalität, den Sitzungen, den Versuchen, dem % von Distraktoren, dem % von Zielen, dem Verzug, dem Signal, dem Stopp oder der Art von Änderung,
und wobei die erhaltenen k Fälle aus Schritt b) die nächsten k Fälle zum neuen Fall des Benutzers sind und unter Verwendung des CBR-Paradigmas ausgewählt werden, welches das Berechnen des Abstands zwischen jedem Fall cᵢ des Satzes von vergangenen Fällen und dem neuen Fall c_{N} umfasst, wobei der Abstand wie folgt berechnet wird: ${d}_{\left({c}_{i}, {c}_{N}\right)} = {d}_{{TDef}_{i , N}} + {d}_{{Prof}_{i , N}} + {d}_{{Conf}_{i , N}} + {d}_{{Res}_{i , N}}$
wobei *d_{TDef_{i,N}}* der Abstand zwischen den Aufgabendefinitionen der Fälle i und N ist, *d_{Prof_{i,N}}* der Abstand zwischen den kognitiven Profilen von Benutzern der Fälle i und N ist, *d_{Conf_{i,N}}* der Abstand zwischen den Aufgabenkonfigurationen der Fälle i und N ist, und *d_{Res_{i,N}}* der Abstand zwischen den Ergebnissen der Fälle i und N ist, und
wobei der Abstand zwischen Aufgabendefinitionen, kognitiven Profilen, Aufgabenkonfigurationen und Ergebnissen als beliebiger Wert definiert wird, welcher jeweils den Unterschied zwischen jeden beiden Aufgabendefinitionen, kognitiven Profilen, Aufgabenkonfigurationen und Ergebnissen definiert, wobei ein oder mehrere Parameter jeder Aufgabendefinition, jedes kognitiven Profils, jeder Aufgabenkonfiguration oder jedes Ergebnisses verwendet werden, um jeweils solchen Abstand zu berechnen.

2. Computerimplementiertes Verfahren nach Anspruch 1, wobei die erhaltenen k Fälle die nächsten k Fälle zum neuen Fall des Benutzers sind und unter Verwendung des fallbasierten Schließen (CBR)-Paradigmas ausgewählt werden, welches das Berechnen des Abstands zwischen jedem Fall cᵢ des Satzes von vergangenen Fällen und dem neuen Fall c_{N} umfasst, der Abstand zwischen Fällen definiert als beliebiger Wert, welcher den Unterschied zwischen jeden beiden Fällen definiert, wobei ein oder mehrere Parameter jedes Falles verwendet wird, um solchen Abstand zu berechnen.

3. Computerimplementiertes Verfahren nach einem der Ansprüche 1 oder 2, wobei der Abstand zwischen den Aufgabendefinitionen der Fälle i und N wie folgt berechnet wird ${d}_{TDef \left(i, N\right)} = \frac{1}{{D}_{N}} {\sum }_{∀ TDef} \left|{TDef}_{i}-{TDef}_{N}\right|$ wobei *TDefᵢ* und *T_{Def_{N}}* jeweils die normierten Koeffizienten ∈ [0,1] sind, welche jeweils die Parameter der Aufgabendefinition in jedem Fall i und N angeben, und D_{N} ein Normierungsfaktor ist, welcher als Summe der maximalen Unterschiede zwischen den TDef-Werten berechnet wird.

4. Computerimplementiertes Verfahren nach einem der Ansprüche 1 bis 3, wobei der Abstand zwischen den kognitiven Profilen der Benutzer der Fälle i und N wie folgt berechnet wird ${d}_{Prof \left(i, N\right)} = \frac{1}{{D}_{N}} {\sum }_{∀ cp} \left|{cpd}_{{u}_{i}}-{cpd}_{{u}_{N}}\right|$ wobei *cpd_{uᵢ}* und *cpd_{u_{N}}* die normierten Koeffizienten ∈ [0,1] sind, welche jeweils den Grad der Beeinträchtigung des kognitiven Prozesses cp für die Benutzer der Fälle i und N angeben, und D_{N} ein Normierungsfaktor ist, welcher als Summe der maximalen Unterschiede zwischen den cpd-Werten berechnet wird.

5. Computerimplementiertes Verfahren nach einem der Ansprüche 1 bis 4, wobei der Abstand zwischen den Aufgabenkonfigurationen der Fälle i und N wie folgt berechnet wird ${d}_{Conf \left(i, N\right)} = \frac{1}{{D}_{N}} {\sum }_{∀ Conf} \left|{Conf}_{i}-{Conf}_{N}\right|$ wobei *Confᵢ* und *Conf_{N}* die normierten Koeffizienten ∈ [0,1] sind, welche jeweils die Parameter der Aufgabenkonfiguration in jedem Fall i und N angeben, und D_{N} ein Normierungsfaktor ist, welcher als Summe der maximalen Unterschiede zwischen den Conf-Werten berechnet wird.

6. Computerimplementiertes Verfahren nach einem der Ansprüche 1 bis 5, wobei der Abstand zwischen den Ergebnissen der Fälle i und N wie folgt berechnet wird ${d}_{Res \left(i, N\right)} = \frac{1}{{D}_{N}} {\sum }_{∀ Res} \left|{Res}_{i}-{Res}_{N}\right|$ wobei *Resᵢ* und *Res*_{N} die normierten Koeffizienten ∈ [0,1] sind, welche jeweils die Parameter des Aufgabenergebnisses in jedem Fall i und N angeben, und D_{N} ein Normierungsfaktor ist, welcher als Summe der maximalen Unterschiede zwischen den Res-Werten berechnet wird.

7. Computerimplementiertes Verfahren nach einem der Ansprüche 1 bis 6, wobei das erste Mal für den Benutzer, die k Fälle unter Verwendung des fallbasierten Schließen (CBR)-Paradigmas ausgewählt werden, welches das Berechnen des Abstands des kognitiven Profils des Benutzers jedes vergangenen Falles i aus dem Satz von vergangenen Fällen mit dem neuen Benutzer umfasst, wobei die Benutzer der vergangenen Fälle gute Rehabilitationsergebnissen aufweisen, als; ${d}_{\left({u}_{i}, {u}_{N}\right)} = \frac{1}{{D}_{N}} {\sum }_{∀ cp} \left|{cpd}_{{u}_{i}}-{cpd}_{{u}_{N}}\right|$ wobei *cpd_{uᵢ}* und *cpd_{u_{N}}* die normierten Koeffizienten ∈ [0,1] sind, welche jeweils den Grad der Beeinträchtigung des kognitiven Prozesses cp für die Benutzer der Fälle i und N angeben, und D_{N} ein Normierungsfaktor ist, welcher als Summe der maximalen Unterschiede zwischen den cpd-Werten berechnet wird.

8. Computerimplementiertes Verfahren nach einem der Ansprüche 1 bis 7, wobei die vom Benutzer durchzuführende Aufgabe bestimmt wird, indem:
a. ein Satz von kognitiven Rehabilitationsaufgaben erhalten wird,
b. die Eignung jeder Aufgabe aus Schritt a) für das kognitive Profil des Benutzers bestimmt wird,
c. der Satz von Aufgaben aus Schritt a) gemäß der Eignung der Aufgabe für das kognitive Profil des Benutzers aus Schritt b) klassifiziert wird,
d. der klassifizierte Satz von Aufgaben aus Schritt c) oder ein ausgewählter Teilsatz der geeignetsten Aufgaben des klassifizierten Satzes von Aufgaben aus Schritt c) einem Therapeuten bereitgestellt wird,
e. die durchzuführende Aufgabe vom Therapeuten erhalten wird, wobei die Aufgabe eine Aufgabe aus dem Satz oder Teilsatz von Aufgaben ist, welche in Schritt d) dem Therapeuten bereitgestellt wurde,
wobei das Bestimmen der Eignung der Aufgabe das Berechnen des Abstands zwischen der Aufgabe t und dem kognitiven Profil des Benutzers u umfasst, welcher wie folgt berechnet wird: ${d}_{\left(u, t\right)} = \frac{1}{D} {\sum }_{cp ∈ {u}_{cp}} cpd \left(maxPr-{Pr}_{{t}_{cp}}\right)$
wobei cp ein kognitiver Prozess ist, cpd der normierte Koeffizient ∈ [0,1] ist, welcher den Grad der Beeinträchtigung des kognitiven Prozesses cp angibt, *Pr_{t_{cp}}* die Priorität der Aufgabe t für solchen kognitiven Prozess cp ist, maxPr ein festgestellter maximaler Prioritätswert ist, und D ein Normierungsfaktor des Abstands ist, welcher wie folgt berechnet wird: $D = max \left(\left|{u}_{cp}\right|\left|{t}_{cp}\right|\right) maxPr$
wobei u_{cp} der Satz von kognitiven Prozessen ist, welche im Benutzer u beeinträchtigt sind, und t_{cp} der Satz von kognitiven Prozessen ist, welche mit der Aufgabe t in Zusammenhang stehen.

9. Computerimplementiertes Verfahren nach einem der Ansprüche 1 bis 8, wobei die Werte der veränderbaren Parameter jedes Falles mittels Gewichtungsmittelung gemäß dem Ergebnis der Aufgabe gemittelt werden, wobei das Gewicht für jene Fälle mit einem übermäßig guten Ergebnis erhöht wird und das Gewicht für jene Fälle mit einem schlechten Ergebnis verringert wird.

10. Computerimplementiertes Verfahren nach einem der Ansprüche 1 bis 9, wobei der kognitive Prozess ein oder mehrere kognitive Prozesse ist, ausgewählt aus: Daueraufmerksamkeit, selektiver Aufmerksamkeit, geteilter Aufmerksamkeit, Verarbeitungsgeschwindigkeit, Entscheidungsfindung, Hemmungskontrolle, kognitiver Flexibilität und/oder Arbeitsgedächtnis.

11. Computerimplementiertes Verfahren nach einem der Ansprüche 1 bis 10, wobei das CBR-Paradigma das Berücksichtigen des Alters der Benutzer umfasst.

12. Computerimplementiertes Verfahren nach einem der Ansprüche 1 bis 11, wobei das CBR-Paradigma das Berücksichtigen der Diagnose der Benutzer umfasst.

13. Computerimplementiertes Verfahren nach einem der Ansprüche 1 bis 12, wobei die Aufgabe ausgewählt wird und/oder die veränderbaren Parameter bestimmt werden, unter Berücksichtigung diverser Zustände des Benutzers, welche eine Behinderung für den Benutzer sein können, wobei vorzugsweise die Zustände eins oder mehrere der Folgenden umfassen: Gehörlosigkeit, Bewegungsverlangsamung, Bradykinesie oder räumlicher Halbseitenvernachlässigung.

14. Computerprogrammprodukt umfassend Anweisungen, sodass, wenn es von einem Prozessor verarbeitet wird, demjenigen dazu ausbilden, eins der Verfahren nach den Ansprüchen 1 bis 13 durchzuführen.

## Revendications

1. Procédé mis en œuvre par ordinateur pour déterminer les paramètres optimaux pour la progression d'une tâche de rééducation de processus cognitif d'un utilisateur, dans lequel la progression est adaptative et personnalisée, comprenant :
a. recevoir une tâche déterminée à réaliser par l'utilisateur, la tâche comprenant des paramètres modifiables qui affectent la difficulté de la tâche ;
b. recevoir un ensemble de k cas à partir d'un ensemble de cas passés, dans lequel les k cas sont choisis en utilisant un paradigme de raisonnement à partir de cas (RàPC) ;
c. moyenner les valeurs des paramètres modifiables de l'ensemble de k cas reçus dans l'étape b) ;
d. déterminer les paramètres optimaux de la tâche comme les paramètres modifiables moyennés de l'étape c) ; et
e. mettre à jour l'ensemble de cas passés avec les résultats de la tâche réalisée avec les paramètres optimaux de l'étape d) ;
dans lequel les paramètres modifiables comprennent un ou plusieurs paramètres choisis parmi : la durée de la tâche, le temps d'exposition, le temps entre les stimuli, *n-back*, la modalité, les sessions, les essais, le % de distracteurs, le % de cibles, le retard, le signal, l'arrêt ou le type de changement,
et dans lequel les k cas reçus de l'étape b) sont les k cas les plus proches du nouveau cas de l'utilisateur et ils sont choisis en utilisant le paradigme de RàPC qui comprend le calcul de la distance entre chaque cas cᵢ de l'ensemble de cas passés et le nouveau cas c_{N}, la distance étant calculée comme suit : ${d}_{\left({c}_{i}, {c}_{N}\right)} = {d}_{{TDef}_{i , N}} + {d}_{{Prof}_{i , N}} + {d}_{{Conf}_{i , N}} + {d}_{{Res}_{i , N}}$
où *d_{TDef_{i,N}}* est la distance entre les définitions de tâche des cas i et N, *d_{Prof_{i,N}}* est la distance entre les profils cognitifs des utilisateurs des cas i et N, *d_{Confi,N}* est la distance entre les configurations de tâche des cas i et N, et *d_{Res_{i,N}}* est la distance entre les résultats des cas i et N, et
dans lequel la distance entre les définitions de tâche, les profils cognitifs, les configurations de tâche et les résultats est définie comme une valeur arbitraire définissant la différence entre chaque deux définitions de tâche, profils cognitifs, configurations de tâche et résultats, respectivement, dans lequel un ou plusieurs paramètres de chaque définition de tâche, profil cognitif, configuration de tâche ou résultat sont utilisés pour calculer une telle distance, respectivement.

2. Procédé mis en œuvre par ordinateur selon la revendication 1, dans lequel les k cas reçus sont les k cas les plus proches du nouveau cas de l'utilisateur et ils sont choisis en utilisant le paradigme de raisonnement à partir de cas (RàPC) qui comprend le calcul de la distance entre chaque case cᵢ de l'ensemble de cas passés et le nouveau cas c_{N}, la distance entre cases étant définie comme une valeur arbitraire définissant la différence entre chaque deux cas, dans lequel un ou plusieurs paramètres de chaque cas sont utilisés pour calculer une telle distance.

3. Procédé mis en œuvre par ordinateur selon l'une quelconque des revendications 1 ou 2, dans lequel la distance entre les définitions de tâche des cas i et N est calculée comme suit ${d}_{TDef \left(i, N\right)} = \frac{1}{{D}_{N}} {\sum }_{∀ TDef} \left|{TDef}_{i}-{TDef}_{N}\right|$ où *TDefᵢ* et *TDef_{N}* sont les coefficients normalisés ∈ [0, 1] indiquant le paramètre de définition de tâche dans chaque cas i et N, respectivement, et D_{N} est un facteur de normalisation calculé comme la somme des différences maximales entre les valeurs de TDef.

4. Procédé mis en œuvre par ordinateur selon l'une quelconque des revendications 1 à 3, dans lequel la distance entre les profils cognitifs de l'utilisateurs des cas i et N est calculée comme suit ${d}_{Prof \left(i, N\right)} = \frac{1}{{D}_{N}} {\sum }_{∀ cp} \left|{cpd}_{{u}_{i}}-{cpd}_{{u}_{N}}\right|$ où *cpd_{uᵢ}* et *cpd_{u_{N}}* sont les coefficients normalisés ∈ [0, 1] indiquant le dégrée d'atteinte du processus cognitif cp pour les utilisateurs des cas i et N, respectivement, et D_{N} est un facteur de normalisation calculé comme la somme des différences maximales entre les valeurs de cpd.

5. Procédé mis en œuvre par ordinateur selon l'une quelconque des revendications 1 à 4, dans lequel la distance entre les configurations de tâche des cas i et N est calculée comme suit ${d}_{Conf \left(i, N\right)} = \frac{1}{{D}_{N}} {\sum }_{∀ Conf} \left|{Conf}_{i}-{Conf}_{N}\right|$ où *Confᵢ* et *Conf_{N}* sont les coefficients normalisés ∈ [0, 1] indiquant le paramètre de configuration de tâche dans chaque cas i et N, respectivement, et D_{N} est un facteur de normalisation calculé comme la somme des différences maximales entre les valeurs de Conf.

6. Procédé mis en œuvre par ordinateur selon l'une quelconque des revendications 1 à 5, dans lequel la distance entre les résultats des cas i et N est calculée comme suit ${d}_{Res \left(i, N\right)} = \frac{1}{{D}_{N}} {\sum }_{∀ Res} \left|{Res}_{i}-{Res}_{N}\right|$ où *Resᵢ* et *Res_{N}* sont les coefficients normalisés ∈ [0, 1] indiquant le paramètre de résultat de tâche dans chaque cas i et N, respectivement, et D_{N} est un facteur de normalisation calculé comme la somme des différences maximales entre les valeurs de Res.

7. Procédé mis en œuvre par ordinateur selon l'une quelconque des revendications 1 à 6, dans lequel dans la première fois pour l'utilisateur, les k cas sont choisis en utilisant le paradigme de raisonnement à partir de cas (RàPC) qui comprend le calcul de la distance du profil cognitif de l'utilisateur de chaque cas passé i à partir de l'ensemble de cas passés avec le nouvel utilisateur, dans lequel les utilisateurs des cas passés présentent des bons résultats de rééducation comme suit ${d}_{\left({u}_{i}, {u}_{N}\right)} = \frac{1}{{D}_{N}} {\sum }_{∀ cp} \left|{cpd}_{{u}_{i}}-{cpd}_{{u}_{N}}\right|$ où *cpd_{uᵢ}* et *cpd_{u_{N}}* sont les coefficients normalisés ∈ [0, 1] indiquant le dégrée d'atteinte du processus cognitif cp pour les utilisateurs des cas i et N, respectivement, et D_{N} est un facteur de normalisation calculé comme la somme des différences maximales entre les valeurs de cpd.

8. Procédé mis en œuvre par ordinateur selon l'une quelconque des revendications 1 à 7, dans lequel la tâche à réaliser par l'utilisateur est déterminer comme suit :
a. recevoir un ensemble de tâches de rééducation cognitive,
b. déterminer l'adéquation de chaque tâche de l'étape a) pour le profil cognitif de l'utilisateur,
c. classer l'ensemble de tâches de l'étape a) en fonction de l'adéquation de la tâche pour le profil cognitif de l'utilisateur de l'étape b),
d. fournir l'ensemble classé de tâches de l'étape c) ou un sous-ensemble sélectionné des tâches les plus appropriées de l'ensemble classé de tâches de l'étape c) à un thérapeute,
e. recevoir la tâche à réaliser à partir du thérapeute, dans lequel la tâche est une tâche de l'ensemble ou du sous-ensemble de tâches fourni dans l'étape d) au thérapeute,
dans lequel la détermination de l'adéquation de la tâche comprend le calcul de la distance entre la tâche t et le profil cognitif de l'utilisateur u, calculée comme suit : ${d}_{\left(u, t\right)} = \frac{1}{D} {\sum }_{cp ∈ {u}_{cp}} cpd \left(maxPr-{Pr}_{{t}_{cp}}\right)$
où cp est un processus cognitif, cpd est le coefficient normalisé ∈ [0, 1] indiquant le dégrée d'atteinte du processus cognitif cp, *Pr_{t_{cp}}* est la priorité de la tâche t pour un tel processus cognitif cp, maxPr est une valeur de priorité maximale établie, et D est un facteur de normalisation de distance, calculé comme suit : $D = max \left(\left|{u}_{cp}\right|\left|{t}_{cp}\right|\right) maxPr$
où u_{cp} est l'ensemble de processus cognitifs atteints chez l'utilisateur u, et t_{cp} est l'ensemble de processus cognitifs liés à la tâche t.

9. Procédé mis en œuvre par ordinateur selon l'une quelconque des revendications 1 à 8, dans lequel les valeurs des paramètres modifiables de chaque cas sont moyennés par moyennage pondérée en fonction du résultat de la tâche, en augmentant le poids pour les cas avec un résultat excessivement bon, et en réduisant le poids pour les cas avec un mauvais résultat.

10. Procédé mis en œuvre par ordinateur selon l'une quelconque des revendications 1 à 9, dans lequel le processus cognitif est un ou plusieurs processus cognitifs choisis parmi : attention soutenue, attention sélective, attention divisée, vitesse de traitement, prise de décisions, contrôle inhibiteur, flexibilité cognitive et/ou mémoire de travail.

11. Procédé mis en œuvre par ordinateur selon l'une quelconque des revendications 1 à 10, dans lequel le paradigme de RàPC comprend la prise en compte de l'âge des utilisateurs.

12. Procédé mis en œuvre par ordinateur selon l'une quelconque des revendications 1 à 11, dans lequel le paradigme de RàPC comprend la prise en compte du diagnostic des utilisateurs.

13. Procédé mis en œuvre par ordinateur selon l'une quelconque des revendications 1 à 12, dans lequel la tâche est choisie et/ou les paramètres modifiables sont déterminés en tenant compte de diverses conditions de l'utilisateur qui peuvent être un handicap pour l'utilisateur, de préférence dans lequel les conditions comprennent une ou plusieurs de : la surdité, la lenteur motrice, la bradycinésie ou l'héminégligence spatiale.

14. Produit de programme informatique comprenant des instructions telles que, lorsqu'elles sont traitées par un processeur, elles le configurent pour réaliser l'un quelconque des procédés selon les revendications 1 à 13.
